# EUROPÄISCHE PATENTSCHRIFT

(11) **EP 0 935 440 B1**
(45) Veröffentlichungstag und Bekanntmachung des Hinweises auf die Patenterteilung: **15.09.2004**
(21) Anmeldenummer: 97949877.1
(22) Anmeldetag: 03.11.1997
(51) Int. Cl.: A61B 5/00, H04B 1/69, A61N 1/372

(54) **ÜBERTRAGUNGSVERFAHREN ZUR DRAHTLOSEN KOMMUNIKATION MIT EINEM IMPLANTIERTEN MEDIZINISCHEN GERÄT**
METHOD FOR WIRELESS COMMUNICATION TRANSFER WITH AN IMPLANTED MEDICAL DEVICE
PROCEDE DE COMMUNICATION SANS FIL AVEC UN APPAREIL MEDICAL IMPLANTE

(30) Priorität: 01.11.1996 DE 19646746
(43) Veröffentlichungstag der Anmeldung: 18.08.1999
(73) Patentinhaber: Nanotron Technologies GmbH, 10555 Berlin (DE)
(72) Erfinder: KOSLAR, Manfred, D-10629 Berlin (DE)
(74) Vertreter: Eisenführ, Speiser & Partner
(86) Internationale Anmeldenummer: PCT/DE1997/002590
(87) Internationale Veröffentlichungsnummer: WO 1998/019591

(56) Entgegenhaltungen:
- US-A- 4 255 791
- US-A- 5 070 500
- US-A- 5 105 294
- US-A- 5 113 278
- US-A- 5 325 394
- US-A- 5 381 798
- KOWATSCH ET AL.: "Spread-Spectrum-übertragung analoger Signale mit Chirp-Modulation" ARCHIV FÜR ELEKTRONIK UND ÜBERTRAGUNGSTECHNIK, Bd. 36, Nr. 7/8, Juli 1982, WÜRZBURG, Seiten 299-304, XP002061685

## Beschreibung

Die Erfindung betrifft ein Verfahren gemäß dem Oberbegriff des Anspruchs 1 und eine Anordnung zur Durchführung des Verfahrens.

Ein derartiges mit einem HF-Trägersignal arbeitendes Verfahren ist beispielsweise aus der deutschen Offenlegungsschrift DE 196 01 866 bekannt.

Ein Verfahren mit den Merkmalen des Oberbegriffs ist aus US-A 5,105,294 bekannt. Aus Kowatsch et al "Spread-Spectrum-Übertragung analoger Signale mit Chirp-Modulation", Archiv für Elektronik und Übertragungstechnik, Bd. 36, Nr. 7/8, Juli 1982, Würzburg, Seiten 299-304, ist ein Verfahren zur Pulskompression mit linearer Frequenzmodulation bekannt. Das Ausgangssignal eines Optimalfilters im Empfänger, die Autokorrelationsfunktion der linearen Frequenzmodulation ist ein Impuls mit einer (sinus x) / x - ähnlichen Einhüllenden.

Es sind verschiedene Verfahren und Vorrichtungen bekannt, um Signale zwischen einem, insbesondere implantierten, medizinischen Gerät und einem externen Sender oder Empfänger zu übertragen. So können beispielsweise moderne Herzschrittmacher über die Schrittmacherelektroden ein intrakardiales Elektrogramm (IEKG) erfassen und zu diagnostischen Zwecken über eine Telemetrieeinheit an ein extrakorporales Kontrollgerät übertragen.

Bei den modernen Signalübertragungsverfahren, wie sie für implantierbare Herzschrittmacher, beispielsweise aus dem Buch von John G. Webster (Editor): "Design of Cardiac Pacemakers", Abschnitt 12 "External Programming", IEEE Press Book Series, New York 1995, bekannt sind, wird das drahtlos zu übertragende digitale Signal im Sender durch einen Modulator in Bitsequenzen dem hochfrequenten Trägersignal aufmoduliert und über eine Übertragungsstrecke dem Empfänger übermittelt, der zur Rückgewinnung des Nachrichtensignals einen entsprechenden Demodulator aufweist. Das Trägersignal liegt dabei in einem verhältnismäßig niedrigen Frequenzbereich, da es den Körper durchdringen muß und auch benachbarte medizinische Geräte nicht stören darf.

Des weiteren besteht bei allen derartigen Verfahren der Nachteil, daß die Qualität des empfängerseitig zurückgewonnenen Nachrichtensignals mit der Entfernung zwischen Empfänger und Sender und mit Störungen auf der Übertragungsstrecke stark abnimmt.

Um bei einer Nachrichtenübertragung auf einer störungsbehafteten Übertragungsstrecke eine gewünschte Reichweite mit einer vorgegebenen Störsicherheit zu erreichen, darf die Sendeleistung deshalb einen bestimmten Wert nicht unterschreiten.

Zum einen hat die somit erforderliche große Sendeleistung den Nachteil, daß der Energieverbrauch während des Sendebetriebs entsprechend hoch ist, was bei batteriebetriebenen Geräten, wie den eingangs erwähnten Herzschrittmachern, wegen der raschen Batterieerschöpfung nachteilig ist. Zum anderen bestehen Befürchtungen, daß die von dem Sender ausgehehde elektromagnetische Strahlung zu einer Schädigung des menschlichen Körpers führen kann, was gerade bei implantierten medizinischen Geräten wegen des extrem geringen Abstandes zum Patienten zu berücksichtigen ist.

Der Erfindung liegt die Aufgabe zugrunde, ein Verfahren der eingangs genannten Art bzw. eine Anordnung zur Durchführung des Verfahrens zu schaffen, welches bei medizinischen implantaten - bei im übrigen mindestens gleichbleibender Übertragungsqualität - eine Verringerung der Sendeleistung bzw. eine Erhöhung der Reichweite-ermöglicht.

Diese Aufgabe wird, ausgehend von einem Verfahren gemäß dem Oberbegriff des Anspruchs 1, durch dessen kennzeichnende Merkmale bzw.-hinsichtlich der Anordnung zur Durchführung des Verfahrens - durch die Merkmale des Anspruchs 14 gelöst.

Die Erfindung schließt die technische Lehre ein, die mit der Nachricht mittels eines bekannten Verfahrens der Nachrichtentechnik modulierten Impulse im Sender einer Winkelmodulation zu unterziehen (was hier als Oberbegriff für Phasen- und Frequenzmodulation zu verstehen ist). Diese winkelmodulierten Impulse werden im Empfänger zeitlich dadurch komprimiert, daß mittels geeigneter Maßnahmen eine frequenzabhängige Verzögerung erfolgt, so daß sich die Zeitdauer der.Impulse verkürzt und diese eine Amplitudenüberhöhung erfahren. Diese Impulskompression läßt sich insbesondere mittels eines Dispersionsfilters durchführen. Aus diesen so bearbeiteten Impulsen läßt sich daraufhin durch entsprechende Demodulation die Nachricht zurückgewinnen, wobei die Demodulation mit einem durch die Amplitudenheraufsetzung verbesserten Signal-/Rauschverhältnis erfolgen kann. Die eigentliche Nachricht kann den Impulsen durch Impulsmodulationsverfahren aufgeprägt werden oder aber dadurch, daß die Impulskompression für zeitlich aufeinanderfolgende Impulse in erkennbar unterschiedlicher Weise vorgenommen wird, so daß diese Variation der Winkelmodulation die Nachricht enthält.

Damit steht nach der Demodulation ein Signal zur Verfügung, welches andernfalls nur mit einer erhöhten Sendeleistung zu gewinnen wäre, wenn nicht ein anderes aufwendiges Verfahren zur Empfangsverbesserung verwendet wird - wie vorzugsweise Diversity-Empfang oder eine Signalkodierung, welche durch redundante Anteile ein größeres Frequenzband oder eine längere Übertragungsdauer in Anspruch nimmt, so daß der zur Verfügung stehende Nachrichtenkanal einen geringeren Informationsdurchsatz aufweist bzw. nur durch eine geringere Zahl von Teilnehmern genutzt werden kann.

Gemäß der Erfindung erfolgt die Winkelmodulation der Impulse im Sender entsprechend einer Modulation, die im Falle einer Frequenzmodulation die Frequenzänderung und im Falle einer Phasenmodulation die Phasenänderung während der Impulsdauer bestimmt. Phasen- und Frequenzmodulation werden hier meist unter dem gemeinsamen Oberbegriff "Winkelmodulation" abgehandelt.

Während die Modulation der Impulse insgesamt unterschiedlichen Impulsmodulationsverfahren folgen kann, wird für die variable Winkelmodulation eine besondere Winkelmodulations-Zeitcharakteristik verwendet, welche sozusagen einer "Modulationskennlinie" entspricht.

Die Modulationskennlinie - hier allgemein Modulationscharakteristik genannt - bestimmt hierbei den zeitlichen Verlauf der Frequenz jeweils während der Dauer eines Impulses. So nimmt die Frequenz des übertragenen Signals vorzugsweise bei einer linear fallenden Modulationscharakteristik jeweils während der Dauer eines Impulses von einem oberhalb der Trägerfrequenz liegenden Wert linear auf einen unterhalb der Trägerfrequenz liegenden Wert ab. Das empfängerseitige Filter ist der verwendeten Modulationscharakteristik durch ein entsprechendes differentielles frequenzabhängiges Laufzeitverhalten in der Weise angepaßt, daß die erzeugten Signalanteile unterschiedlicher Phasenlage zu einem nahezu koinzidenten Signal überlagert werden.

Die Aufprägung der zu übertragenden Nachricht erfolgt wahlweise durch Änderung bzw. Auswahl der Modulationscharakteristik oder durch ein anderes herkömmliches Modulationsverfahren, welches auf die Signallaufzeit ohne oder nur von untergeordnetem Einfluß ist. Bevorzugt kommt hier die Änderung der Amplitude des übertragenen Signals in Abhängigkeit vom Eingangssignal - also eine Amplitudenmodulation oder jede Art von Codierung, bei der die übertragene Information durch die Art, Zahl, Position oder Folge der übermittelten Impulse bestimmt wird - in Betracht.

Mit der Erfindung besteht auf vorteilhafte Weise die Möglichkeit, Signale zu, insbesondere implantierten, Vorrichtungen auch auf höheren Frequenzen als bisher üblich zu übertragen, ohne daß einerseits eine Beeinträchtigung des Gewebes und anderseits eine elektromagnetische Störbeeinflussung (EMI) anderer in der klinischen Umgebung verwendeter Geräte erfolgt. Dies war bisher ein Hauptproblem bei der Benutzung von elektromagnetische Wellen ausstrahlenden Geräten im klinischen Bereich überhaupt. Diese Bedingungen verboten bisher beispielsweise auch die Benutzung von tragbaren Telefonen etc. Darüber hinaus bietet das erfindungsgemäße Verfahren den Vorteil, daß eine Signalübertragung auch über größere Entfernungen (beispielsweise innerhalb eines Krankenzimmers) erfolgen kann, so daß dem Patienten Programmiergeräte etc. nicht unmittelbar auf den Körper aufgelegt zu werden brauchen. Auch ist es bei der Auswahl entsprechender Codes möglich, daß mit verschiedenen Geräte parallel kommuniziert wird, ohne daß eine gegenseitige Störbeeinflussung auftritt. Da die verwendeten Signale mit niedriger Amplitude gesendet werden können, übersteigen sie den umgebenden Rauschpegel nicht oder nur unwesentlich. Damit ist auch die gegenseitige Beeinflussung gering.

In einer bevorzugten Ausführungsform der Erfindung erfolgt die Aufprägung der Informationen des Eingangssignals dadurch, daß die Modulationscharakteristik in Abhängigkeit vom Eingangssignal ausgewählt bzw. verändert wird. Weist das Eingangssignal einen High-Pegel auf, so wird beispielsweise eine mit dem Signal linear fallende Modulationscharakteristik verwendet, was zu einem frequenzmodulierten Impuls mit einer während der Impulsdauer abnehmenden Frequenz führt. Bei einem Low-Pegel des Eingangssignals wird dagegen eine linear steigende Modulationscharakteristik verwendet, was entsprechend zu einem Impuls mit einer während der Impulsdauer zunehmenden Frequenz führt. Die empfängerseitigen Filtermittel sind entsprechend angepaßt.

Die Erfindung ist jedoch nicht auf lineare Modulationscharakteristiken beschränkt, sondern läßt sich vielmehr mit beliebig geformten Modulationskennlinien realisieren, wobei es lediglich erforderlich ist, den verschiedenen Pegeln des Eingangssignals unterschiedliche Modulationen zuzuordnen, um eine anschließende Signalunterscheidung im Empfänger zu ermöglichen.

Auch ist es möglich, mehr als zwei Modulationscharakteristiken für das Eingangssignal zu verwenden, um mit jedem Impuls einen größeren Informationsanteil zu übertragen. Stehen vorzugsweise vier verschiedene Modulationscharakteristiken zur Auswahl, so können entsprechend vier unterschiedliche Impulse übertragen werden, was einer Informationsmenge von jeweils 2 Bit bei jedem übertragenen Impuls entspricht. Durch die Erhöhung der Anzahl unterschiedlicher Modulationscharakteristiken läßt sich also vorteilhaft die Datenübertragungsrate steigern, wobei jedoch zu beachten ist, daß die frequenzmodulierten Impulse mit sehr großer Zahl verschiedener Modulationscharakteristiken immer schwieriger zu unterscheiden sind, was die Störanfälligkeit der Übertragung erhöht.

In der vorstehend beschriebenen Variante der Erfindung erfolgt die Modulation der Impulse aktiv sowohl für einen High-Pegel als auch für einen Low-Pegel des digitalen Eingangssignals. Dies bedeutet, daß sowohl bei einem High-Pegel als auch bei einem Low-Pegel des Eingangssignals frequenzmodulierte Impulse erzeugt werden, die sich durch die Frequenzänderung während der Impulsdauer unterscheiden. Die Aufprägung der in dem Eingangssignal enthaltenen Nachricht auf das übertragene Signal erfolgt hierbei also durch die Auswahl bzw. Änderung der Modulationscharakteristik in Abhängigkeit vom Eingangssignal.

In einer anderen Variante der Erfindung erfolgt die Winkelmodulation der Impulse im Sender dagegen unabhängig von dem zu übertragenden Eingangssignal entsprechend einer einzigen vorgegebenen Modulationscharakteristik, welche die zeitliche Änderung der Frequenz bzw. Phasenlage jeweils während der Dauer eines Impulses bestimmt. Die Aufprägung der in dem Eingangssignal enthaltenen Nachricht auf das übertragene Signal kann hierbei entsprechend den an sich bekannten digitalen Modulationsverfahren auf verschiedene Arten erfolgen. So ist es vorzugsweise günstig, eine Pulspositionsmodulation (PPM), auch Pulsabstandsmodulation genannt - durchzuführen, bei der die Lage der einzelnen frequenzmodulierten Impulse in Abhängigkeit vom Eingangssignal geändert wird.

In einer bevorzugten Ausführungsform der Erfindung erfolgt die Aufprägung der in dem Eingangssignal enthaltenen Nachricht auf das übertragene Signal dagegen durch Pulscodemodulation (PCM), in dem die Folge der zu übertragenden Impulse in Abhängigkeit vom Eingangssignal geändert wird. Bei einem digitalen Eingangssignal erfolgt die Übertragung des Eingangssignals beispielsweise nur bei einem Pegel aktiv, während bei dem anderen Pegel kein Impuls erzeugt wird, so daß sich die verschiedenen Impulse nur durch ihre Amplitude unterscheiden. So wird vorzugsweise bei einem High-Pegel des Eingangssignals ein linear ansteigend frequenzmodulierter Impuls erzeugt, während bei einem Low-Pegel eine Pause mit der Länge eines Impulses eingelegt wird. Diese Variante der Erfindung ermöglicht vorteilhaft eine Realisierung mit nur einer Modulationscharakteristik zur Modulation der Impulse des digitalen Eingangssignals.

Die Erfindung ist in dieser Ausgestaltung zur Aufprägung der in dem Eingangssignal enthaltenen Nachricht auf das übertragene Signal jedoch nicht auf die vorstehend beispielhaft erwähnte Pulspositions- bzw. Pulscodemodulation beschränkt, sondern läßt sich prinzipiell mit allen bekannten digitalen Modulationsverfahren realisieren.

Das auf eine der vorstehend beschriebenen Arten frequenzmodulierte Signal wird dann von dem Sender über die Ubertragungsstrecke zu einem Empfänger übertragen und dort demoduliert, um das Nachrichtensignal zurückzugewinnen.

Der Begriff Übertragungsstrecke ist hierbei und im folgenden allgemein zu verstehen und umfaßt alle drahtlosen übertragungsstrecken, bei denen die Informationsübertragung vom Sender zum Empfänger mittels elektromagnetischer Wellen erfolgt.

Um die senderseitig erzeugten frequenzmodulierten Impulse im Empfänger besser von Störsignalen unterscheiden zu können, werden diese Impulse im Empfänger komprimiert, was zu einer entsprechenden Amplitudenerhöhung führt, indem der Signal-/Rausch-Abstand vergrößert wird.

Ein weiterer Vorteil des erfindungsgemäßen Verfahrens ist in dem wesentlich geringeren Störpotential gegenüber anderen Sendern bzw. Empfängern zu sehen, da ein vorgegebenes Signal/Rausch-Verhältnis nach der Impulskompression im Empfänger mit einer geringeren Sendeleistung erreichbar ist. Darüber hinaus führen die geringeren Anforderungen an die Sendeleistung vorteilhaft zu einer verringerten Umweltbelastung durch elektromagnetische Strahlung.

Zur Kompression der empfängerseitig aufgenommenen Impulse, die entsprechend der senderseitig verwendeten Modulationscharakteristik frequenzmoduliert sind, wird das empfangene Signal mittels eines Dispersionsfilters mit einem vorgegebenen frequenzabhängigen differentiellen Laufzeitverhalten gefiltert.

In der zuvor beschriebenen Variante der Erfindung mit nur einer einzigen Modulationscharakteristik zur senderseitigen Erzeugung der frequenzmodulierten Impulse ist empfängerseitig auch nur ein Dispersionsfilter erforderlich, wobei das frequenzabhängige Laufzeitverhalten dieses Dispersionsfilters derart an die Modulationscharakteristik der senderseitig vorgenommenen Winkelmodulation angepaßt ist, daß die spektralen Signalanteile des senderseitig erzeugten frequenzmodulierten Impulses im wesentlich koinzident am Ausgang des Dispersionsfilters erscheinen, was zu einer Impulskompression und einer entsprechenden Amplitudenerhöhung führt. Erfolgt die senderseitige Winkelmodulation vorzugsweise entsprechend einer linear fallenden Modulationscharakteristik, so nimmt die Frequenz des Impulses während der Impulsdauer ab, was zur Folge hat, daß die höherfrequenten Signalanteile empfängerseitig vor den niederfrequenten Signalanteilen erscheinen. Das Laufzeitverhalten des empfängerseitigen Dispersionsfilters muß diesen "Vorsprung" der höherfrequenten Signalanteile deshalb ausgleichen, damit sich die spektralen Signalanteile des frequenzmodulierten Impulses am Ausgang des Dispersionsfilters zu einem Impuls mit erhöhter Amplitude überlagern.

Die Rückgewinnung der in dem Eingangssignal enthaltenen Nachricht erfolgt anschließend durch einen dem Dispersionsfilter nachgeschalteten Detektor, der an das Modulationsverfahren angepaßt ist, das senderseitig zur Aufprägung der in dem Eingangssignal enthaltenen Nachricht verwendet wird.

Wird dagegen senderseitig in Abhängigkeit von der Amplitude des Eingangssignals eine von mehreren Modulationscharakteristiken ausgewählt, vorzugsweise eine linear fallende Modulationscharakteristik bei einem High-Pegel und eine linear steigende Modulationscharakteristik bei einem Low-Pegel des Eingangssignals, so bestehen zur Auswertung im Empfänger grundsätzlich zwei Möglichkeiten.

Eine Möglichkeit besteht darin, empfängerseitig nur ein Dispersionsfilter vorzusehen, dessen Laufzeitverhalten an eine der senderseitig verwendeten Modulationscharakteristiken in der weise angepaßt ist, daß die spektralen Signalanteile des entsprechend dieser Modulationscharakteristik frequenzmodulierte Impulses am Ausgang des Dispersionsfilters im wesentlichen koinzident erscheinen, was zu einer Impulskompression und Amplitudenerhöhung führt. Erfolgt die Frequenzmodulation senderseitig dagegen entsprechend einer der anderen Modulationscharakteristiken, die nicht optimal an das Laufzeitverhalten des empfängerseitigen Dispersionsfilters angepaßt ist, so erscheinen die spektralen Signalanteile des frequenzmodulierten Impulses zeitlich verteilt am Ausgang des Dispersionsfilters und somit wegen der geringeren Impulskompression oder auch -expansion auch mit einer geringeren Amplitude. In dieser Ausführungsform hängt die Amplitude des am Ausgang des Dispersionsfilters erscheinenden Impulses also von der senderseitig verwendeten Modulationscharakteristik und damit von der Amplitude des Eingangssignals ab, die zur Auswahl der Modulationscharakteristik herangezogen wird. Um das digitale Eingangssignal aus dem Ausgangssignal des Dispersionsfilters zurückzugewinnen ist, dem Dispersionsfilter deshalb ein Detektor nachgeschaltet, der beispielsweise als Amplitudendemodulator ausgeführt sein kann.

Die andere Möglichkeit sieht dagegen vor, den frequenzmodulierten Impuls empfängerseitig mehreren Dispersionsfiltern zuzuführen. Das differentielle Laufzeitverhalten der empfängerseitig angeordneten Dispersionsfilter und die senderseitig verwendeten Modulationscharakteristiken sind hierbei jeweils paarweise derart aneinander angepaßt, daß die spektralen Signalanteile des frequenzmodulierten Impulses am Ausgang genau eines der Dispersionsfilter im wesentlichen koinzident erscheinen und dadurch zu einer Amplitudenerhöhung führen, während die Ausgangssignale der anderen Dispersionsfilter wegen der abweichenden Charakteristik nicht erhöht sind. Das Eingangssignal kann also danach diskriminiert werden, an welchem der Dispersionsfilter eine Amplitudenerhöhung vorliegt.

Als Dispersionsfilter werden vorteilhafterweise Oberflächenwellenfilter (engl. SAW-Filter - Surface Acoustic Waves) verwendet. Ein Dispersionsfilter weist hierbei ein frequenzabhängiges differentielles Laufzeitverhalten auf, das an die senderseitig vorgenommene Winkelmodulation derart angepaßt ist, daß die verschiedenen Spektralanteile des übertragenen Signals im Empfänger aufgrund ihrer unterschiedlichen Laufzeit durch das Dispersionsfilter an dessen Ausgang quasi koinzident erscheinen, so daß die Ausgangsamplitude durch optimale Superposition der Spektralanteile stark erhöht wird.

Erfindungsgemäß wird zunächst näherungsweise ein (quasi-)Dirac-Impuls erzeugt und einem Tiefpaßfilter zugeführt, dessen Filterkennlinie kurz vor Erreichen der Grenzfrequenz eine Überhöhung aufweist und den Stoß-Impuls somit in einen si-Impuls (Spaltimpuls) transformiert, dessen Form durch die bekannte si-Funktion si (x)=^{sinx}/ₓ beschrieben wird. Das si-förmige Ausgangssignal des Tiefpaßfilters wird anschließend auf einen Amplitudenmodulator gegeben, der der Trägerschwingung eine si-förmige Hüllkurve aufprägt. Wird das auf diese Weise erzeugte Signal einem dispergierenden Filter zugeführt, so erscheint am Ausgang ein frequenzmodulierter Impuls. In der Erfindung erfolgt also senderseitig zunächst eine Expansion des relativ scharfen si-Impulses durch das Dispersionsfilter in einen frequenzmodulierten Impuls, der gegenüber dem si-Impuls verlängert ist und eine entsprechend geringere Amplitude aufweist. Empfängerseitig erfolgt dann ebenfalls durch ein Dispersionsfilter wieder eine Kompression des Impulses mit einer entsprechenden Amplitudenerhöhung. Da zur senderseitigen Expansion und empfängerseitigen Kompression der Impulse jeweils ein Dispersionsfilter verwendet wird, eignet sich die Erfindung vorteilhaft für einen Transceiverbetrieb mit abwechselndem Sende- und Empfangsbetrieb. Hierzu können Sender und Empfänger jeweils korrespondierende baugleiche Baugruppen mit jeweils einem Dispersionsfilter aufweisen, die im Sendebetrieb zur Erzeugung des frequenzmodulierten Impulses und im Empfangsbetrieb zur Kompression der empfangenen frequenzmodulierten Impulse dienen.

Bei dem erfindungsgemäßen Nachrichtenübertragungssystem ist zur Erreichung einer Impulskompression im Empfänger eine Anpassung des frequenzabhängigen Laufzeitverhaltens des empfängerseitig verwendeten Dispersionsfilters an die Modulationscharakteristik der senderseitig vorgenommenen Frequenzmodulation erforderlich.

In einer Variante der Erfindung werden hierzu jeweils aneinander angepaßte Sender-Empfänger-Paare hergestellt, so daß bei der Inbetriebnahme keine weiteren Abstimmarbeiten erforderlich sind. Die vorstehend erwähnten Dispersionsfilter werden hierbei bevorzugt als Oberflächenwellenfilter (SAW-Filter: Surface Acoustic Waves) realisiert, da sich derartige Filter mit hoher Genauigkeit und Stabilität herstellen lassen. Darüber hinaus bieten derartige Oberflächenwellenfilter den Vorteil, daß sich Amplitudengang und Phasengang unabhängig voneinander dimensionieren lassen, was die Möglichkeit eröffnet, das in jedem Empfänger erforderliche schmalbandige Bandpaßfilter und das Dispersionsfilter in einem Bauteil zu verwirklichen. Derartige Filter sind für andere Anwendungsbereiche beispielsweise aus der europäischen Patentanmeldung EP 0 0223 554 A2 bekannt.

In einer anderen Variante der Erfindung wird der Empfänger dagegen vor oder während des Betriebs durch Veränderung des Laufzeitverhaltens des empfängerseitig verwendeten Dispersionsfilters an den Sender angepaßt.

Es ist deshalb gemäß einer vorteilhaften Variante der Erfindung vorgesehen, daß der Sender im Rahmen eines Anpassungsvorgangs ein Referenzsignal erzeugt, das vorzugsweise einer Folge von High-Pegeln des Eingangssignals entspricht, wobei die Modulationscharakteristik der senderseitig vorgenommenen Frequenzmodulation oder das frequenzabhängige Laufzeitverhalten des empfängerseitigen Dispersionsfilters so lange verändert wird, bis empfängerseitig eine optimale Impulskompression bzw. Amplitudenerhöhung auftritt. Besonders vorteilhaft ist diese Variante bei der Verwendung eines digitalen Signalprozessors zur Filterung und Aufbereitung im Empfänger, da ein derartiger Signalprozessor in einfacher Weise eine Änderung des frequenzabhängigen Laufzeitverhaltens und eine entsprechende Optimierung ermöglicht, wobei der Optimierungsvorgang rechnergesteuert automatisch ablaufen kann.

In einer weiteren vorteilhaften Ausführungsform dieser Variante erfolgt die Datenübertragung blockweise, wobei der vorstehend beschriebene Anpassungsvorgang bei jedem Block erneut durchgeführt wird, um Schwankungen der Dispersionseigenschaften der Übertragungsstrecke dynamisch ausgleichen zu können.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1a, 1b: als bevorzugtes Ausführungsbeispiel der Erfindung einen Sender bzw. einen Empfänger zur Datenübertragung von einem implantierten medizinischen Gerät an ein extrakorporales Kontrollgerät,
- Figur 2a bis 2e: das digitale Eingangssignal des Senders sowie verschiedene Zwischenstadien der Signalverarbeitung im Sender bis zum Sendesignal sowie
- Figur 3a bis 3d: das Empfangssignal sowie verschiedene Zwischenstadien der Signalverarbeitung im Empfänger bis zum demodulierten Signal,
- Figur 4a, 4b: Sender bzw. Empfänger eines derartigen Nachrichtenübertragungssystems mit aktiver Übertragung von High- und Low-Pegeln in einer Darstellung als Blockschaltbild,
- Figur 5a bis 5k: das digitale Eingangssignal des Senders in Figur 4a sowie verschiedene Zwischenstadien der Signalverarbeitung im Sender,
- Figur 6a bis 6e: das empfängerseitig aufgenommene Signal sowie mehrere Zwischenstadien der Signalverarbeitung im Empfänger sowie
- Figur 7, 8: jeweils eine abgewandelte Form des in Figur 4b dargestellten Empfängers mit einer Rauschunterdrückungsschaltung.

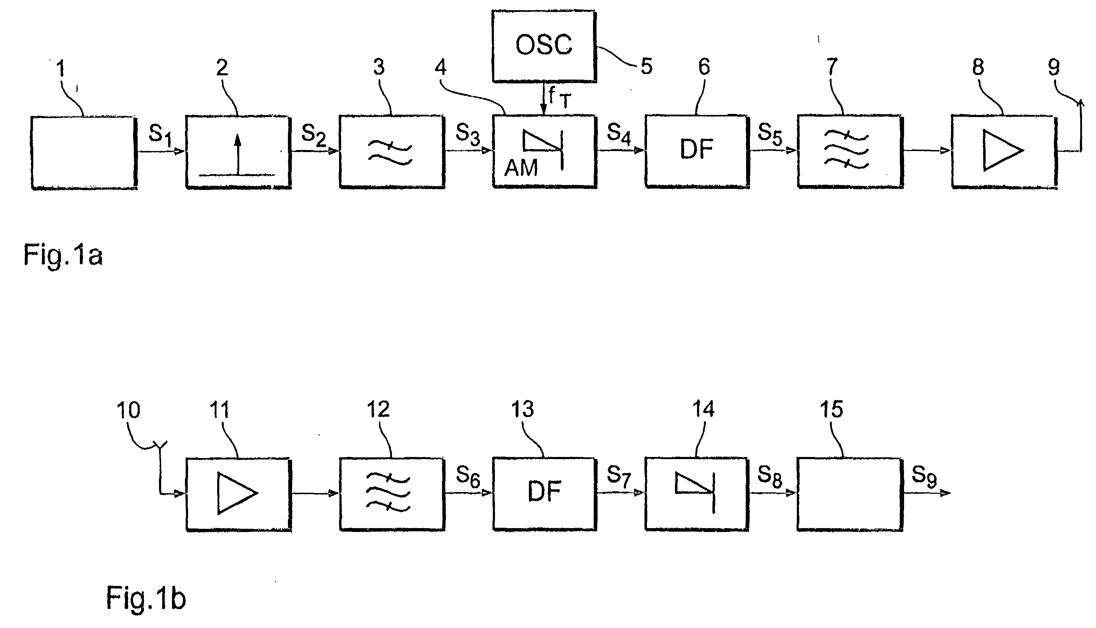

Der in Figur 1a dargestellte Sender dient zur Datenübertragung von einem implantierten medizinischen Gerät an ein extrakorporales Kontrollgerät. So ist es beispielsweise möglich, über die Elektroden eines Herzschrittmachers ein intrakardiales Elektrokardiogramm (IEKG) zu erfassen und an ein extrakorporales Kontrollgerät zu übertragen, welches das IEKG auf einem Bildschirm darstellt und zu diagnostischen Zwecken einer weiteren Signalverarbeitung unterzieht. Der in Figur 1a dargestellte Sender zeichnet sich für diese Anwendung in Verbindung mit dem in Figur 1b dargestellten Empfänger vorteilhaft dadurch aus, daß die Übertragung bei vorgegebenen Anforderungen an Reichweite und Störsicherheit vorteilhaft mit einer relativ geringen Sendeleistung erfolgen kann, was zum einen die Batterielebensdauer erhöht und zum anderen die Umweltbelastung durch elektromagnetische Strahlung - auch als Elektro-Smog bezeichnet - verringert. Darüber hinaus weist der Sender aufgrund der relativ geringen Sendeleistung ein verringertes Störpotential anderen Nachrichtenübertragungssystemen gegenüber auf.

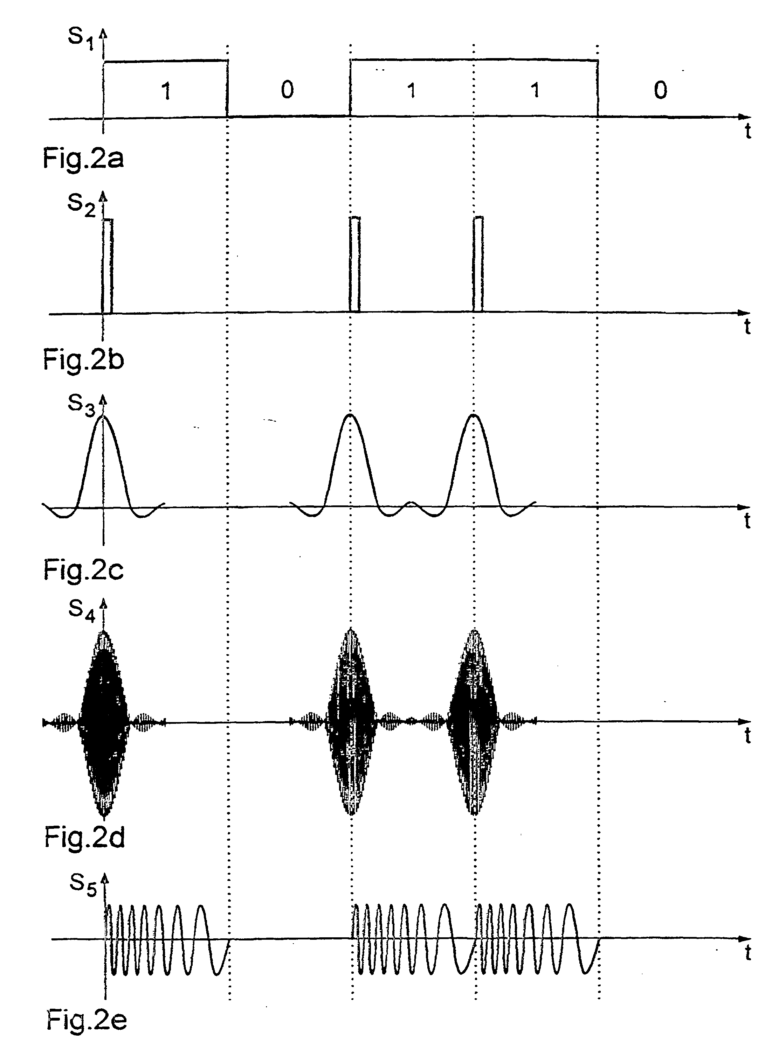

Ein digitales Eingangssignal s₁, das beispielsweise durch Digitalisierung des IEKG-Signals gewonnen wird und dessen zeitlicher Verlauf in Figur 2a detailliert dargestellt ist, wird im Sender zunächst einem Pulsformer 2 zugeführt, der die relativ breiten Rechteckimpulse des Eingangssignals s₁ in kurze Nadelimpulse transformiert, die (quasi-)Dirac-Impulse nachbilden sollen. Aus der Darstellung der Nadelimpulsfolge s₂ in Figur 2b ist ersichtlich, daß die Erzeugung der einzelnen Nadelimpulse jeweils durch die steigende Flanke eines Rechteckimpulses des Eingangssignals s₁ getriggert wird.

Eine auf diese Weise erzeugte Nadelimpulsfolge s₂ wird anschließend einem Tiefpaßfilter 3 zugeführt, dessen Laufzeitverhalten kurz vor der Grenzfrequenz eine Überhöhung aufweist, so daß die einzelnen Nadelimpulse - wie aus Figur 2c ersichtlich - jeweils in si-Impulse transformiert werden, deren Form der bekannten si-Funktion (Spaltfunktion) si(x)=^{sin x}/ₓ entspricht.

Im Anschluß daran wird die si-Impulsfolge s₃ einem Amplitudenmodulator 4 (oder -multiplikator) zugeführt, der dieses Signal auf eine Trägerschwingung der Frequenz f_{T} aufmoduliert, die von dem Oszillator 5 erzeugt wird, so daß am Ausgang des Amplitudenmodulators 4 - wie in Figur 2d dargestellt - Trägerfrequenzimpulse mit einer si-förmigen Hüllkurve erzeugt werden. (Die Impulse sind in der Zeichnung aus darstellungstechnischen Gründen verbreitert dargestellt, sind also - bei maßstäblicher Darstellung - tatsächlich schmaler.)

Dem Amplitudenmodulator 4 ist ein Dispersionsfilter 6 nachgeschaltet, welches das modulierte Trägerfrequenzsignal s₄ entsprechend seiner frequenzabhängigen differentiellen Laufzeitcharakteristiken filtert. Am Ausgang des Dispersionsfilters 6 erscheinen deshalb - wie aus Figur 2e ersichtlich - linear frequenzmodulierte Impulse mit konstanter Amplitude, deren Frequenz während der Impulsdauer von einem oberhalb der Trägerfrequenz f_{T} liegenden Wert f_{T}+Δf/2 auf einen unterhalb der Trägerfrequenz f_{T} liegenden Wert f_{T}-Δf/2 abfällt.

Bei dem hier dargestellten Sender erfolgt die Übertragung des Eingangssignals s₁ also unipolar, d.h. es wird lediglich bei einem High-Pegel des Eingangssignals s₁ ein Sendeimpuls erzeugt, während ein Low-Pegel an einer Pause des Sendesignals s₅ erkennbar ist. Aus diesem Grund lassen sich Sender und Empfänger vorteilhaft relativ einfach mit jeweils nur einem Dispersionsfilter 6 bzw. 13 aufbauen.

Die auf diese Weise erzeugte Impulsfolge s₅ wird nachfolgend einem Bandpaßfilter 7 zugeführt, dessen Mittenfrequenz gleich der Trägerfrequenz f_{T} der frequenzmodulierten Impulse ist, so daß außerhalb des Übertragungsbandes liegende Signale ausgefiltert werden. Das bandpaßbegrenzte Signal wird schließlich über einen Sendeverstärker 8 der Antenne 9 zugeführt und abgestrahlt.

Der in Figur 1b dargestellte Empfänger ermöglicht den Empfang des von dem vorstehend beschriebenen Sender abgestrahlten linear frequenzmodulierten Signals sowie die Demodulation und Rückgewinnung des digitalen Eingangssignals s₃ bzw. s₁.

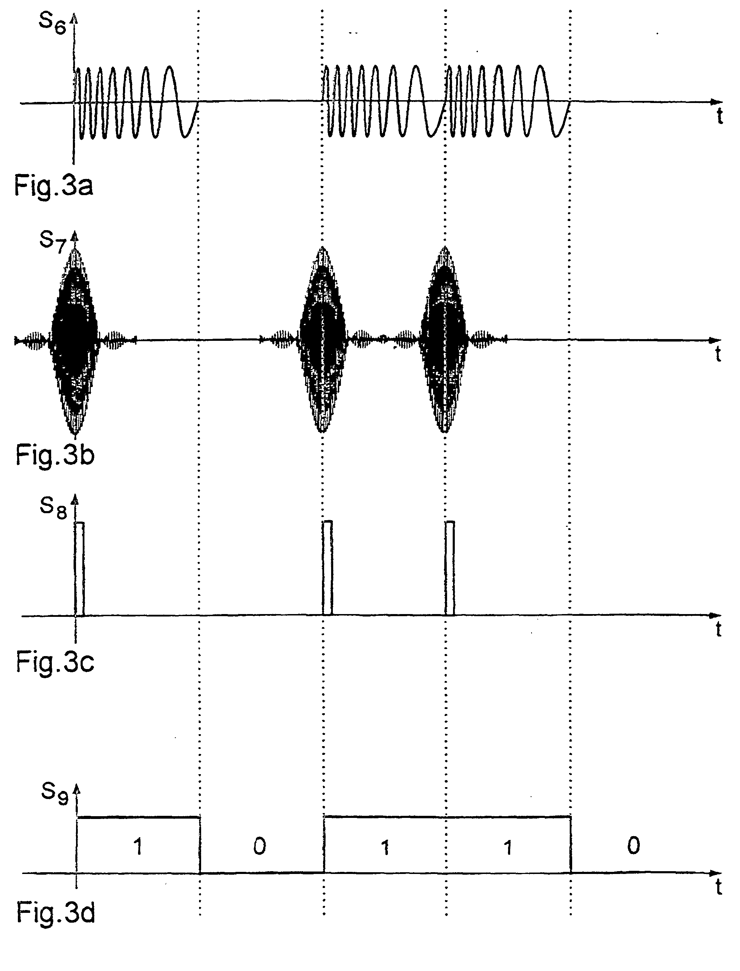

Hierzu wird bei dem vorliegenden Ausführungsbeispiel das über die Empfängerantenne 10 - beispielsweise auch im Diversity-Betrieb - empfangene Signal zunächst einem Vorverstärker 11 und nachfolgend einem Bandpaßfilter 12 zugeführt, dessen Mittenfrequenz gleich der Trägerfrequenz f_{T} des bandpaßbegrenzten Sendesignals ist, so daß Störsignale aus anderen Frequenzbereichen aus dem empfangenen Signal ausgefiltert werden. (Anstelle eines konventionellen Bandpaßfilters kann hier auch vorteilhafterweise ein oder das nachfolgende SAW-Filter verwendet werden.) Der zeitliche Verlauf des auf diese Weise aufbereiteten Signals s₆ ist detailliert in Figur 3a dargestellt, wobei zur Vereinfachung eine störungsfreie Übertragungsstrecke angenommen wird.

Das empfangene Signal s₆ besteht also aus einer Folge von linear frequenzmodulierten Impulsen, wobei die Frequenz entsprechend der senderseitig verwendeten Modulationscharakteristik während der Impulsdauer linear von einem oberhalb der Trägerfrequenz liegenden Wert f_{T}+Δf/2 auf einen unterhalb der Trägerfrequenz f_{T} liegenden Wert f_{T}-Δf/2 abfällt.

Das Signal s₆ wird anschießend einem Dispersionsfilter 13 zugeführt, das die einzelnen Impulse des Eingangssignals s₆ zeitlich komprimiert, was zu einer entsprechenden Amplitudenerhöhung und damit einem verbesserten Signal-/Rauschabstand führt.

Die Impulskompression nutzt hierbei die Tatsache, daß die höherfrequenten Signalanteile der Impulse aufgrund der senderseitig durchgeführten linearen Frequenzmodulation am Eingang des Dispersionsfilters 13 vor den niederfrequenten Signalanteilen erscheinen. Das Dispersionsfilter 13 gleicht deshalb den "Vorsprung" der höherfrequenten Signalanteile aus, indem diese in dem Dispersionsfilter stärker verzögert werden als die niederfrequenten Signalanteile. Das frequenzabhängige differentielle Laufzeitverhalten des Dispersionsfilters 13 ist hierbei derart an die Modulationscharakteristik der senderseitig durchgeführten Frequenzmodulation angepaßt, daß die spektralen Signalanteile des empfangenen Signals im wesentlichen koinzident am Ausgang des Dispersionsfilters 13 erscheinen und sich somit - wie aus Figur 3b ersichtlich - zu einem Signal s₇ mit jeweils impulsweise si-förmiger Hüllkurve überlagern, wobei die Amplitude der einzelnen Impulse gegenüber dem empfangenen linear frequenzmodulierten Signal s₆ wesentlich vergrößert ist. (An dieser Stelle ist darauf hinzuweisen, daß bei den in den Figuren dargestellten prinzipiellen Signaldarstellungen zur Vergrößerung der Deutlichkeit eine Verzerrung vorgenommen wurde. Die linear frequenzmodulierten Impulse sind in der Realität im Verlauf enger und die komprimierten Signale sehr viel schmaler.

Das Ausgangssignal des Dispersionsfilters 13 wird dann einem Demodulator 14 zugeführt, der das Signal s₇ von der hochfrequenten Trägerschwingung befreit und - wie aus Figur 3c ersichtlich - ein diskretes Ausgangssignal s₈ mit nadelförmigen Impulsen erzeugt.

Im Anschluß daran wird aus den nadelförmigen Impulsen mittels eines Impulsformers 15 das ursprüngliche digitale Signal s₉ zurückgewonnen, dessen zeitlicher Verlauf detailliert in Figur 3d dargestellt ist.

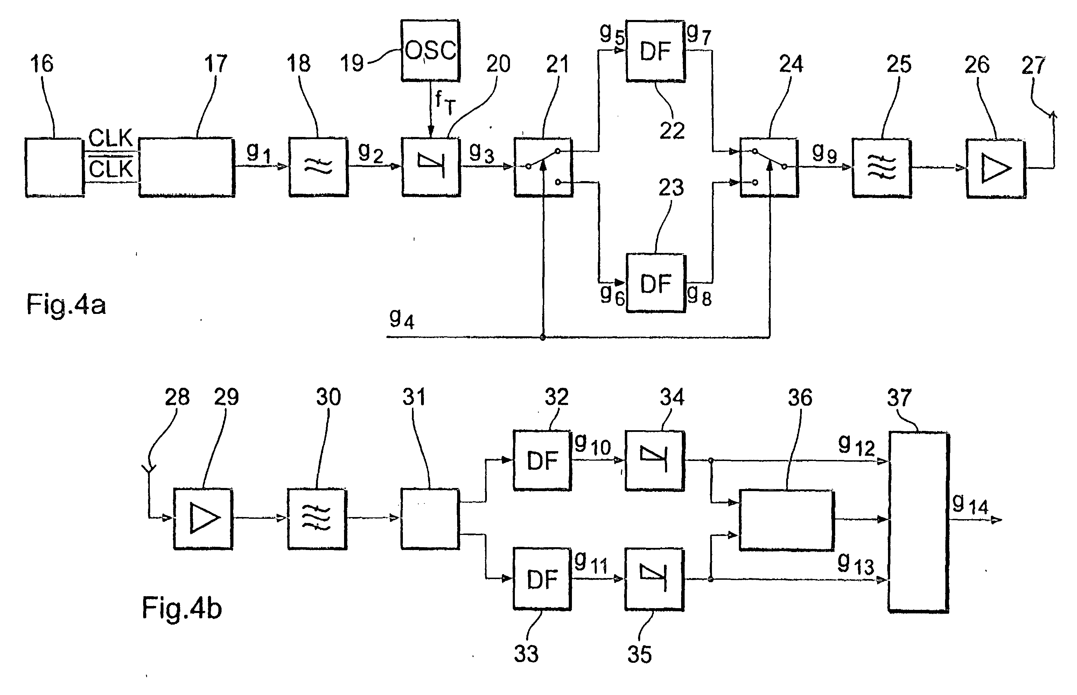

In den Figuren 4a und 4b ist ein weiteres erfindungsgemäß ausgestaltetes Nachrichtenübertragungssystem dargestellt, das sich von dem vorstehend beschriebenen und in den Figuren 1a und 1b dargestellten einfacheren Ausführungsbeispiel im wesentlichen dadurch unterscheidet, daß sowohl der High-Pegel als auch der Low-Pegel des digitalen Nachrichtensignals aktiv übertragen wird, was zu einer erhöhten Störsicherheit beiträgt. Auch dieses Übertragungssystem eignet sich wegen der geringeren Anforderungen an die Sendeleistung insbesondere für die Datenübertragung von einem implantierten medizinischen Gerät an ein extrakorporales Kontrollgerät.

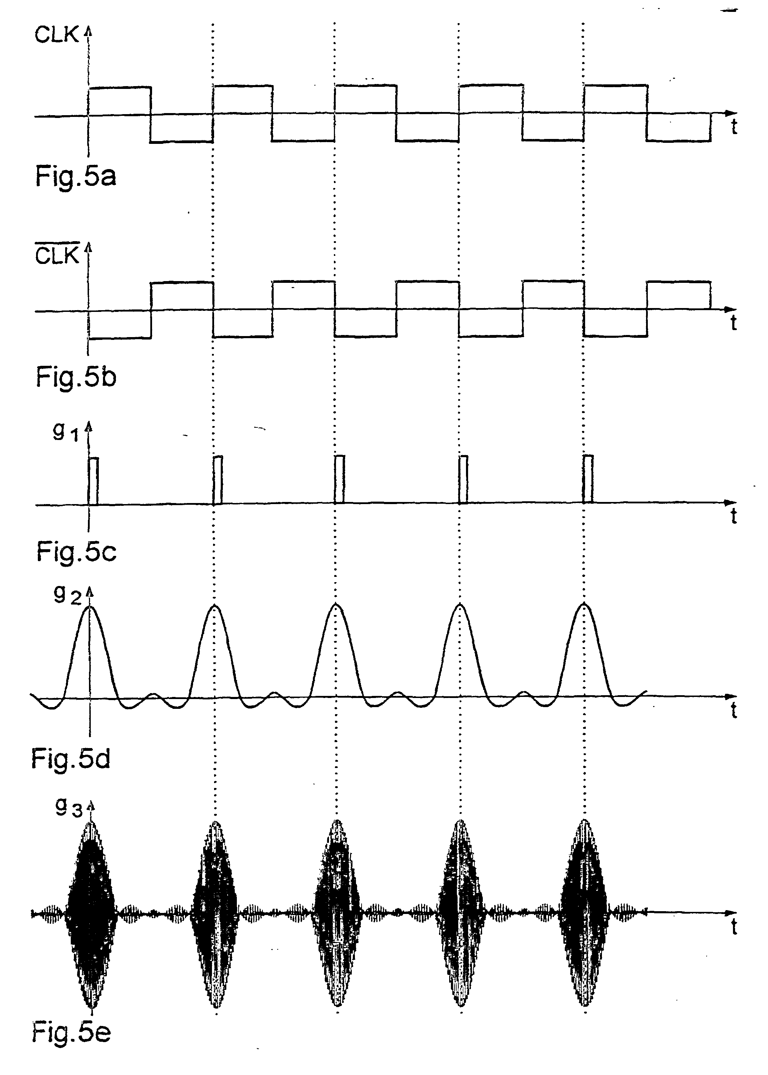

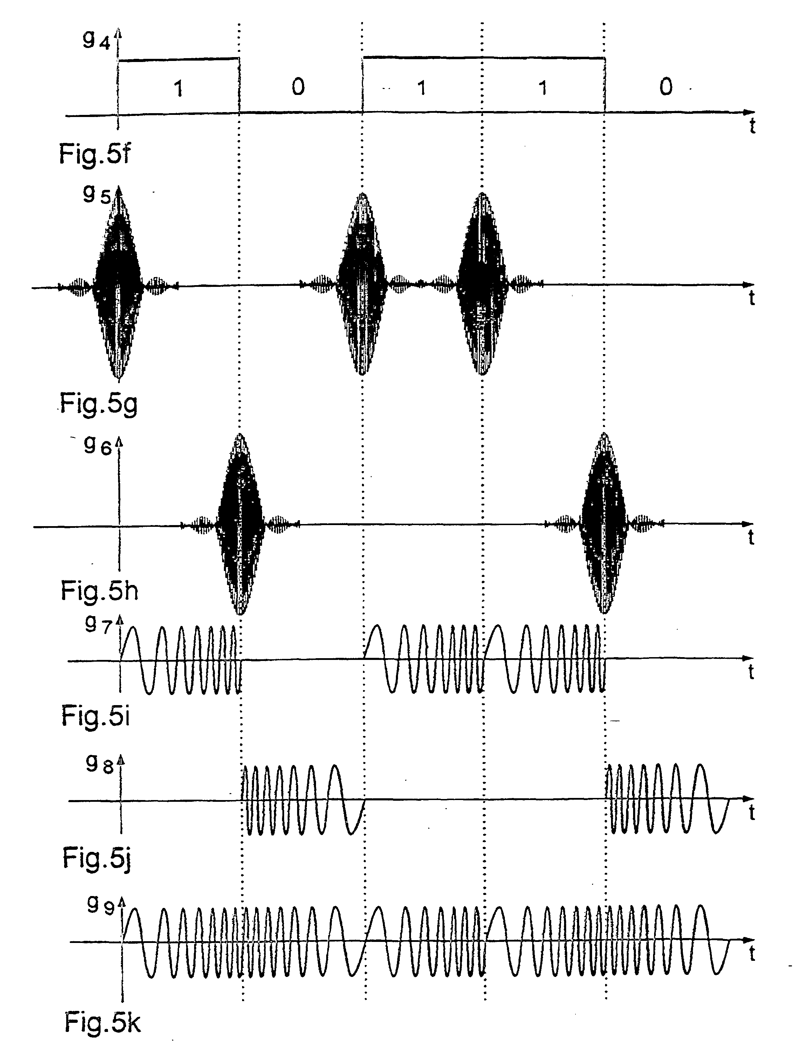

Der in Figur 4a dargestellte Sender weist hierzu einen Pulsformer 17 auf, der von einem Taktgeber 16 mit den in Figur 5a bzw. 5b dargestellten gegenphasigen Taktsignalen angesteuert wird und an seinem Ausgang - wie in Figur 5c dargestellt - eine Folge g₁ von nadelförmigen Impulsen ausgibt, die eine (Quasi-)Dirac-Stoß-Folge nachbilden. Die auf diese Weise erzeugte Impulsfolge g₁ wird anschließend einem Tiefpaßfilter 18 zugeführt, dessen Filterkennlinie kurz vor der Grenzfrequenz eine Überhöhung aufweist und die nadelförmigen Impulse jeweils in si-förmige Impulse (Spaltimpulse) transformiert, die detailliert in Figur 5d dargestellt sind. Nachfolgend wird diese Impulsfolge g₂ mittels eines Amplitudenmodulators 20 auf eine von dem Oszillator 19 erzeugte Trägerschwingung mit der Trägerfrequenz f_{T} aufmoduliert. Am Ausgang des Amplitudenmodulators 20 (oder -multiplikators) erscheint somit eine Folge g₃ von äquidistanten Trägerfrequenzimpulsen mit jeweils si-förmiger Hüllkurve. Wichtig ist in diesem Zusammenhang, daß die am Ausgang des Amplitudenmodulators 20 erscheinende Impulsfolge g₃ unabhängig von dem digitalen Eingangssignal g₄ ist und somit keine Information trägt.

Die Aufprägung der Information des Eingangssignals g₄ erfolgt anschließend mittels eines Analog-Schalters 21, der von dem Eingangssignal g₄ angesteuert wird und die von dem Amplitudenmodulator 20 erzeugte Impulsfolge g₃ in Abhängigkeit von der Amplitude des Eingangssignals g₄ entweder einem Dispersionsfilter 22 mit einer frequenzabhängig linear fallenden Laufzeit oder einem Dispersionsfilter 23 mit einer frequenzabhängig linear steigenden Laufzeit zuführt. Ausgangsseitig sind die beiden Dispersionsfilter 22, 23 mit einem weiteren Analog-Schalter 24 oder einer Summierstufe verbunden, der in Abhängigkeit von der Amplitude des Eingangssignals g₄ das Ausgangssignal g₇ bzw. g₈ eines der beiden Dispersionsfilter 22, 23 auswählt und weiterleitet.

Am Ausgang des Analog-Schalters 24 erscheint somit - wie in Figur 5k dargestellt - eine Folge g₉ von jeweils impulsweise linear frequenzmodülierten Trägerfrequenzimpulsen, wobei die einzelnen Impulse bei einem High-Pegel des Eingangssignals g₄ innerhalb der Impulsdauer eine linear zunehmende Frequenz aufweisen, wohingegen bei einem Low-Pegel des Eingangssignals g₄ die Frequenz innerhalb eines Impulses linear abnimmt.

Das am Ausgang des Analog-Schalters 24 erscheinende Signal wird anschließend von einem Bandpaßfilter 25 gefiltert, um außerhalb des Obertragungsbandes liegende Störsignale zu unterdrücken. Das auf diese Weise gewonnene Signal wird dann von einem Sendeverstärker 26 verstärkt und über die Sendeantenne 27 abgestrahlt.

Figur 4b zeigt den zugehörigen Empfänger, der das von dem in Figur 4a dargestellten Sender abgestrahlte Signal über eine Antenne 28 empfängt und zunächst in einem Vorverstärker 29 verstärkt und in einem Bandpaßfilter 30 von solchen Störsignalen befreit, deren Frequenz außerhalb des Übertragungsbandes liegt.

Anschließend wird das empfangene Signal über ein Koppelelement 31 zwei Dispersionsfiltern 32, 33 zugeführt. Das frequenzabhängige Laufzeitverhalten der beiden empfängerseitigen Dispersionsfilter 32, 33 ist hierbei jeweils paarweise an das frequenzabhängige Laufzeitverhalten der beiden senderseitig angeordneten Dispersionsfilter 22, 23 in der Weise angepaßt, daß sich die spektralen Signalanteile des empfangenen Signals am Ausgang eines der beiden Dispersionsfilter 32 bzw. 33 zu einem Impuls mit erhöhter Amplitude addieren, während am Ausgang des anderen Dispersionsfilters 33 bzw. 32 wegen der Fehlanpassung nur ein abgeschwächter Impuls erscheint.

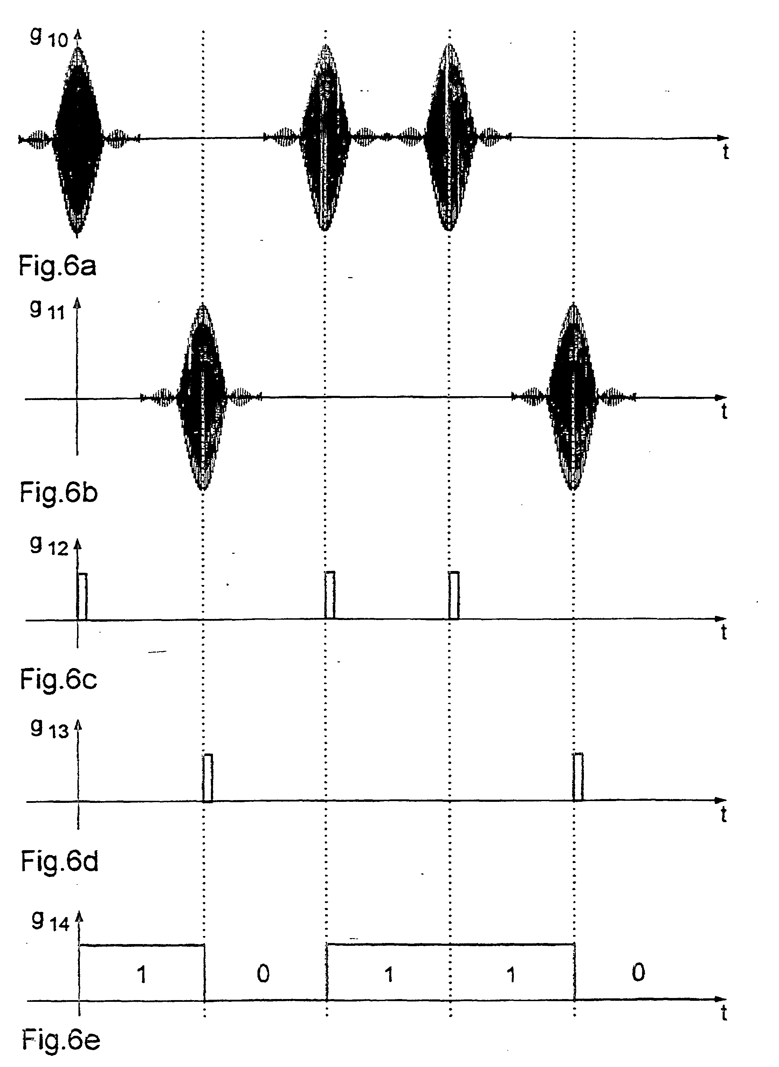

Aus den Figuren 6a und 6b ist ersichtlich, daß die Ausgangssignale g₁₀ bzw. g₁₁ der Dispersionsfilter 32, 33 jeweils aus einer Folge von Trägerfrequenzimpulsen mit einer si-förmigen Hüllkurve bestehen.

Die am Ausgang der beiden Dispersionsfilter 32, 33 erscheinenden Signale g₁₀ bzw. g₁₁ werden anschließend jeweils einem Demodulator 34, 35 zugeführt, der die Signale g₁₀ bzw. g₁₁ von der Trägerschwingung befreit und jeweils nadelförmige Impulse erzeugt, wie aus Figur 6c bzw. 6d ersichtlich ist.

Während die Nadelimpulse am Ausgang des einen Demodulators 34 jeweils einem High-Pegel des Eingangssignals g₄ entsprechend, kennzeichnen die am Ausgang des anderen Demodulators 35 erscheinenden Nadelimpulse jeweils einen Low-Pegel des Eingangssignals g₄.

Um aus diesen beiden Signalen g₁₂, g₁₃ das ursprüngliche Eingangssignal g₄ zurückzugewinnen, werden die beiden Signale g₁₂, g₁₃ zur Triggerung einem Taktgeber 36 zugeführt, der ein Taktsignal erzeugt, welches die Taktrate des ursprünglichen Eingangssignals g₄ wiedergibt. Dieses Taktsignal wird zusammen mit den Ausgangssignalen g₁₂, g₁₃ der beiden Demodulatoren 34, 35 dem Dekoder 37 zugeführt, der das ursprüngliche Ausgangssignale g₄ bzw. g₁₄ zurückgewinnt, wie aus Figur 6e ersichtlich ist.

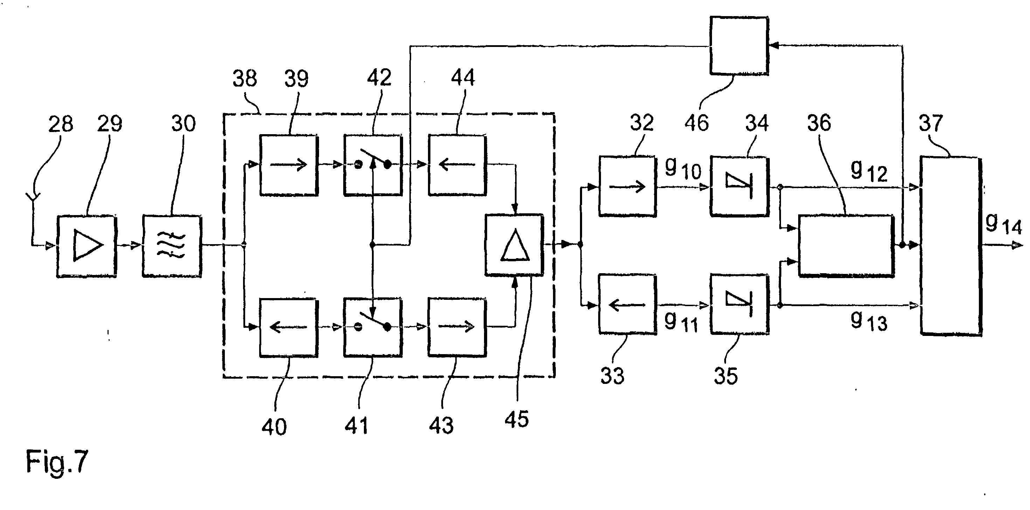

Figur 7 zeigt eine abgewandelte Form des in Figur 4b dargestellten Empfängers mit einer Rauschunterdrückungsschaltung 38, die auch mit anderen Empfängern für derartige Chirp-Signale kombiniert werden kann. Wegen der weitgehenden Übereinstimmung dieses Empfängers mit dem in Figur 4b dargestellten Empfänger sind funktionsgleiche Bauelemente in beiden Figuren mit denselben Bezugszeichen versehen.

Wie bei dem bereits vorstehend beschriebenen Empfänger wird das senderseitig gechirpte Signal über die Antenne 28 aufgenommen und zunächst einem Eingangsverstärker 29 sowie einem Bandpaßfilter 30 zugeführt, das auf die Trägerfrequenz abgestimmt ist und somit außerhalb des Übertragungsbandes liegende Störsignale ausfiltert. Anschließend wird das Signal der Rauschunterdrückungsschaltung 38 zugeführt und von dieser zunächst auf zwei parallele Zweige aufgeteilt, in denen jeweils zwei zueinander inverse Dispersionsfilter 39, 44 bzw. 40, 43 in Reihe geschaltet sind. Bei einer aktiven Übertragung sowohl eines logischen LOW-Pegels als auch eines logischen HIGH-Pegels ist also eines der beiden eingangsseitig angeordneten Dispersionsfilter 39 bzw. 40 derart angepaßt, daß am Ausgang dieses Dispersionsfilters 39 bzw. 40 ein zeitlich komprimierter Impuls erscheint. Am Ausgang des anderen Dispersionsfilters 39 bzw. 40 erscheint dagegen ein auf die doppelte Länge zeitlich expandierter Impuls. Die beiden Analogschalter 41, 42 unterbrechen jedoch den Signalfluß in den beiden Zweigen symmetrisch um die Mitte des komprimierten Impulses, so daß der zeitlich komprimierte Impuls unterdrückt wird und lediglich der zeitlich expandierte Impuls im anderen Zweig übrig bleibt. Die Ansteuerung der Analogschalter 41, 42 erfolgt hierbei durch die Synchronsierungsschaltung 46, die von dem Taktgeber 36 angesteuert wird und somit den Takt des Ausgangssignals und damit den Übertragungstakt wiedergibt. Die nachfolgenden Dispersionsfilter 43, 44 erzeugen aus dem zeitlich expandierten Impuls dann wieder den ursprünglichen Impuls mit der ursprünglichen Länge und entsprechend auch mit der ursprünglichen Amplitude. Diese Impulse werden dann auf den Subtrahierer 45 geleitet, an dessen Ausgang somit im wesentlichen der ursprüngliche Impuls erscheint.

Anders liegen die Verhältnisse dagegen bei dem Rauschen, das durch die störungsbehaftete Übertragungsstrecke verursacht wird und zusammen mit dem Nutzsignal vom Empfänger aufgenommen wird. Dieses Rauschen wird zunächst durch die Dispersionsfilter 39, 40 in unterschiedlicher Richtung verschoben. Die nachgeschalteten Dispersionsfilter 43, 44 machen diese Verschiebung jedoch wieder rückgängig, so daß das Eingangsrauschen in den beiden Zweigen bis auf den sehr kurzen, von den Analogschaltern 41, 42 ausgeschnittenen Teil rekonstruiert wird. Die Differenzbildung durch den Subtrahierer 45 führt deshalb zu einer nahezu weitgehenden Unterdrückung des empfängerseitig aufgenommenen Rauschens.

Die weitere Verarbeitung des derart aufbereiteten Signals erfolgt dann wie in der Beschreibung zu Figur 4b ab der Gabel 31 ausgeführt.

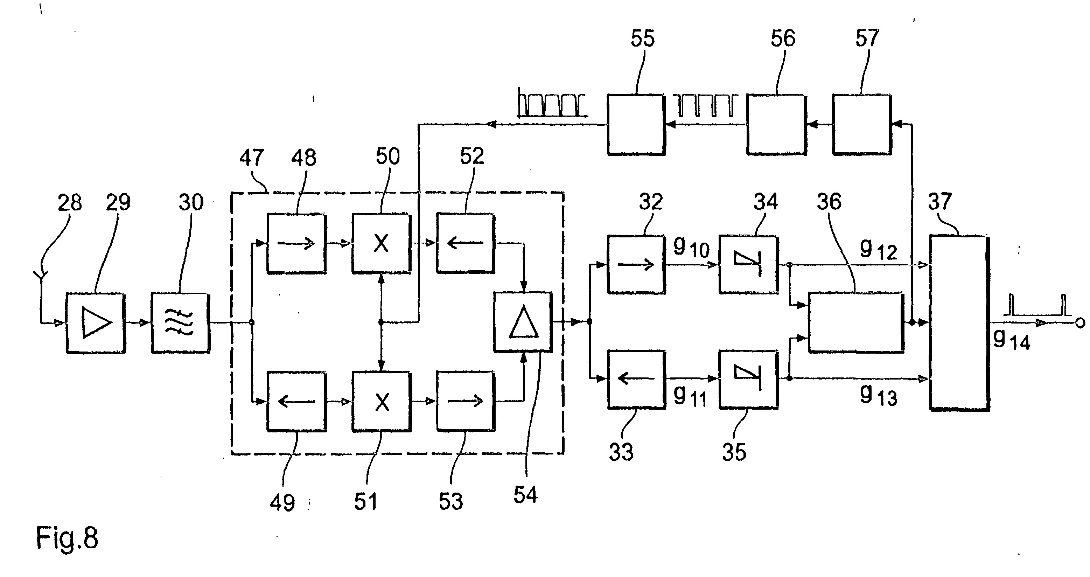

Der in Figur 8 dargestellte Empfänger unterscheidet sich von dem vorstehend beschriebenen und in Figur 7 dargestellten Empfänger im wesentlichen durch den Aufbau und die Ansteuerung der Rauschunterdrückungsschaltung 47. Wegen der somit vorhanden weitgehenden Übereinstimmung der beiden Schaltungen sind funktionsgleiche Bauelemente bzw. Baugruppen in Figur 7 und 8 durch dieselben Bezugszeichen gekennzeichnet.

Wie auch bei dem in Figur 7 dargestellten Empfänger werden die gechirpten Impulse über die Antenne 28 aufgenommen und zunächst einem Eingangsverstärker 29 und einem Bandpaßfilter 30 zugeführt, das auf die Trägerfrequenz abgestimmt ist und somit außerhalb des Übertragungsbandes liegende Störsignale ausfiltert.

Anschließend wird das Signal der Rauschunterdrückungsschaltung 47 zugeführt, die das Signal zunächst auf zwei parallele Zweige aufteilt, in denen jeweils zwei zueinander inverse Dispersionsfilter 48, 52 bzw. 49, 53 in Reihe geschaltet angeordnet sind. Am Ausgang der Rauschunterdrükkungsschaltung 47 werden die beiden Zweige von dem Subtrahierer 54 zusammengeführt, wodurch das Rauschen in dem empfangenen Signal aufgrund der Differenzbildung vollständig unterdrückt wird.

Im Gegensatz dazu wird das gechirpte Signal durch die Differenzbildung im Subtrahierer 54 nicht aufgehoben, so daß sich der Signal/Rauschabstand wesentlich erhöht. Die eingangsseitig angeordneten Dispersionsfilter 48, 49 sind hierbei derart an die senderseitig erzeugten gechirpten Signale angepaßt, daß am Ausgang eines der Dispersionsfilter 48, 49 ein zeitlich komprimierter Impuls mit entsprechend erhöhter Amplitude erscheint, während am Ausgang des anderen Dispersionsfilters 49, 48 ein zeitlich expandierter Impuls mit entsprechend verringerter Amplitude erscheint. Der Signalfluß in den beiden Zweigen wird nun jeweils beim Erscheinen des komprimierten Impulses - wie noch detailliert beschrieben wird - durch die Multiplizierer 50, 51 synchron unterdrückt, so daß der komprimierte Impuls etwa entsprechend seiner Hüllkurve ausgeschnitten wird. Durch die nachgeschalteten Dispersionsfilter 52, 53 wird dann aus dem zeitlich expandierten Impuls wieder der ursprüngliche Impuls erzeugt, so daß am Ausgang des Subtrahierers 54 im wesentlichen das ursprünglich empfangene Signal mit einem wesentlich verbesserten Signal/Rauschabstand erscheint.

Die Ansteuerung der Multiplizierer 50, 51 erfolgt in fester Synchronisation zum Übertragungstakt, um das Signal in den beiden Zweigen der Rauschunterdrückungsschaltung 47 jeweils exakt beim Erscheinen des zeitlich komprimierten Impuls unterdrücken zu können. Hierzu weist der Empfänger eine Synchronisationsschaltung 57 auf, die eingangsseitig zur Synchronisation mit dem Taktgeber 36 verbunden ist. Über einen nachgeschalteten Pulsformer 56 und ein Tiefpaßfilter 55 werden dann invertierte, mit der Spitze zu Null liegende Spaltimpulse mit der Amplitude 1 erzeugt, die den Multiplizierern 50, 51 zugeführt werden. Die Multiplizierer 50, 51 multiplizieren die Signale in den beiden Zweigen der Rauschunterdrückungsschaltung 47 also entweder mit Null oder mit Eins, was entsprechend entweder zu einer Unterdrückung des Signals führt oder das Signal im wesentlichen unverändert passieren läßt. Die Multiplizierer 50, 51 haben hier also die gleiche Wirkung wie die Schaltelemente 41, 42 in der zuvor beschriebenen Variante der Rauschunterdrükkungsschaltung 38.

## Patentansprüche

1. Verfahren zur drahtlosen Kommunikation mit einem, insbesondere in den menschlichen Körper implantierten, medizinischen Gerät, wobei ein Nachrichten-Eingangssignal (s₁, g₄) in einem Sender (2 bis 8, 16 bis 26) einer Winkelmodulation, d.h. einer Phasen- oder Frequenzmodulation, unterworfen wird und über einen Übertra gungskanal zu einem Empfänger (11 bis 15, 29 bis 37) gelangt,
wobei
im Sender die Nachricht tragende, ein Frequenzspektrum aufweisende, winkelmodulierte Impulse (s₅, g₉) derart erzeugt werden, daß diese im Empfänger (11 bis 15, 29 bis 37) mittels eines Filters mit frequenzabhängiger Laufzeit, insbesondere eines Dispersionsfilters (13, 32, 33) derart zeitlich komprimierbar sind, daß Impulse mit gegenüber den ausgesandten Impulsen verkürzter Dauer und erhöhter Amplitude entstehen, und die Impulse senderseitig mindestens einen Teil der die Nachricht ausmachenden Information nach einem weiteren-Modulations-oder Codierungsverfahren der Nachrichtentechnik aufgeprägt erhalten oder mindestens ein Teil der die Nachricht ausmachenden Information zusätzlich der Winkelmodulation aufgeprägt ist,
und wobei
im Sender zunächst näherungsweise eine Quasi-Dirac-Impulsfolge (s₂, g₁) erzeugt und einem Tiefpaßfilter (3, 18) zugeführt wird, dessen Filterkennlinie kurz vor Erreichen der Grenzfrequenz eine Überhöhung aufweist und die Stoß-Impulsfolge somit in eine Folge von si-Impulsen bzw. Spaltimpulsen (s₃, g₂) transformiert, deren Form durch die si-Funktion si (x) =^{sinx}/ₓ beschrieben wird, welche anschließend auf einen Amplitudenmodulator (4, 20) gegeben wird, der einer Trägerschwingung (f_{T}) eine impulsweise si-förmige Hüllkurve aufprägt, und
das auf diese Weise erzeugte Signal (s₄, g₃) einem dispergierenden Filter (6; 22, 23) zugeführt wird, an dessen Ausgang eine frequenzmodulierte Impulsfolge erscheint.

2. Verfahren nach Anspruch 1, **dardurch gekennzeichnet,** daß
die Impulsfolge entsprechend einem vorgegebenen Filterverhalten gefiltert wird, wobei die senderseitige Winkelmodulation und das empfängerseitige frequenzabhängige differentielle Laufzeitverhalten des Dispersionsfilters (13, 32, 33) derart aufeinander abgestimmt sind, daß die spektralen Signalanteile der winkelmodulierten Impulsfolge (s₆) des Ausgangssignals (s₉, g₁₄) aufgrund der frequenzabhängig unterschiedlichen Signallaufzeit des Dispersionsfilters (13, 32, 33) an dessen Ausgang im wesentlichen koinzident und mit entsprechender Amplitudenerhöhung erscheinen.

3. Verfahren nach Anspruch 1, **dadurch gekennzeichnet, daß**
es sich bei dem weiteren Modulationsverfahreh um eine Pulspositionsmodulation (PPM) oder eine Pulscodemodulation (PCM) oder eine differentielle Pulscodemodulation (DPCM) oder eine Pulsdeltamodulation (PDM) oder eine Modifikation eines oder mehrerer dieser Modulationsverfahren handelt.

4. Verfahren nach Anspruch 3, **dadurch gekennzeichnet, daß**
die Winkelmodulation und das weitere Modulationsverfahren voneinander unabhängige, orthogonale oder angenähert orthogonale Modulationsarten bilden.

5. Verfahren nach Anspruch 1, **dadurch gekennzeichnet, daß**
das Eingangssignal (g₄) eine Trägerfrequenz aufweist, die im Sender (16 bis 26) jeweils impulsweise der Winkelmodulation unterworfen wird.

6. Verfahren nach Anspruch 2, **dadurch gekennzeichnet, daß**
die winkelmodulierten Impulse im Empfänger (29 bis 37) mindestens zwei Dispersionsfiltern (32, 33) zugeführt werden, wobei das unterschiedliche Laufzeitverhalten der Dispersionsfilter (32, 33) und die senderseitig verwendeten Modulationscharakteristiken jeweils paarweise derart aneinander angepaßt sind, daß die spektralen Signalanteile der frequenzmodulierten Impulse am Ausgang nur eines der beiden Dispersionsfilter (32, 33) im wesentlichen koinzident und mit entsprechender Amplitudenerhöhung erscheinen, während bei dem jeweils anderen Dispersionfilter (32, 33) diese Kompression nicht stattfindet.

7. Verfahren nach Anspruch 1, **dadurch gekennzeichnet, daß**
die Amplitude der von dem Dispersionsfilter (13) komprimierten Impulse mittels eines Detektors (14, 15) ausgewertet wird.

8. Verfahren nach Anspruch 1, **dadurch gekennzeichnet, daß**
die Winkelmodulation entsprechend einer vorgegebenen Modulationscharakteristik erfolgt, welche die zeitliche Änderung des Phasenwinkels jeweils während der Dauer eines Impulses nach einem vorbestimmten zeitvariablen Ablauf bestimmt, daß in dem Amplitudenmodulator (4) die Amplitude der winkelmodulierten Impulse zur Aufprägung der im Eingangssignal (si) enthaltenen Nachricht, in Abhängigkeit vom Eingangssignal (s₁) vorgegeben wird,
daß die winkelmodulierten Impulse im Empfänger (11 bis 15) einem Dispersionsfilter (13) zugeführt werden, dessen Laufzeitverhalten derart mit einem reversen zeitvariablen Ablauf an die senderseitig verwendete Modulationscharakteristik der Winkelmodulation angepaßt ist, däß die spektralen Signalanteile, der winkelmodulierten Impulse (s₆) im wesentlichen koinzident und mit einer entsprechenden Amplitudenerhöhung am Ausgang des Dispersionsfilters (13) erscheinen,
daß die Amplitude der von dem Dispersionsfilter (13) komprimierten Impulse zur Rückgewinnung der in dem Eingangssignal (s₁) enthaltenen Nachricht mittels eines Detektors (14, 15), insbesondere eines Amplitudendemodulators, ausgewertet wird.

9. Verfahren nach Anspruch 8, **dadurch gekennzeichnet, daß**
sich während der Pulsdauer der pulsmodulierten Signale der Winkel - die Frequenz oder die Phase - der Trägerfrequenz linear oder nichtlinear nach einem vorgegebenen Profil mit der Zeit innerhalb der Pulsdauer von einer unteren Frequenz oder Phasenlage zu einer oberen Frequenz oder Phasenlage bzw. umgekehrt monoton ändert, wobei der Empfänger ein entsprechend komplementäres Verhalten der Dispersionsfilter aufweist.

10. Verfahren nach Anspruch 9, **dadurch gekennzeichnet, daß**
das vorgegebene Profil sich innerhalb der Impulsfolge - jeweils im Verhältnis einzelner Impulse untereinander - verändert, wobei diese Veränderung ebenfalls einen Teil der Nachricht bildet.

11. Verfahren nach Anspruch 1, **dadurch gekennzeichnet,**
**daß** zur Anpassung von Sender (2 bis 8, 16 bis 26) und Empfänger (11 bis 15, 29 bis 37) während eines Anpassungsvorgangs zum Abgleich ein vorgegebenes digitales Referenzsignal als Eingangssignal (s₁, g₄) übertragen wird,
**daß** während des Anpassungsvorgangs die Amplitude oder die Impulsdauer des Ausgangssignals (s₇, g₁₀, g₁₁) des empfängerseitigen Dispersionsfilters (13, 32, 33) gemessen und die senderseitig verwendete Modulationscharakteristik oder das frequenzabhängige Laufzeitverhalten des empfängerseitigen Dispersionsfilters (13, 32, 33) verändert wird, bis die Impulsdauer am Ausgang der Dispersionsfilter im Empfänger einen Minimalwert bzw. die Amplitude einen Maximalwert annimmt.

12. Verfahren nach Anspruch 1, **dadurch gekennzeichnet,**
**daß** der Signalfluß im Empfänger in zwei parallele Zweige mit jeweils zwei Dispersionsfiltern (39, 44, 40, 43) mit zueinander inversen frequenzabhängigen Laufzeitcharakteristiken aufgeteilt wird,
**daß** der Signalfluß in den beiden Zweigen jeweils während eines Impulses für einen vorgegebenen Zeitraum durchgeschaltet oder unterbrochen wird, wobei die Unterbrechung bzw. Durchschaltung synchron zum übertragungstakt erfolgt,
**daß** die beiden Zweige ausgangsseitig durch einen Subtrahierer (45) zusammengeführt werden.

13. Verfahren nach Anspruch 1, **dadurch gekennzeichnet, daß**
der Frequenzbereich des Trägersignals im Bereich von 400 MHz und 1 GHz liegt.

14. Sender- und Empfänger-Anordnung zur Durchführung des Verfahrens nach einem der vorhergehenden Ansprüche, mit
einem Sender (2 bis 8, 16 bis 26) zur Aufnahme und Übertragung eines Eingangssignals (s₁, g₄), der zur Winkelmodutation, d.h. einer Phasen- oder Frequenzmodulation, des Eingangssignals (s₁, g₄) einen ersten Modulator (2 bis 6, 16 bis 24) aufweist sowie
einem Empfänger (11 bis 15, 29 bis 37), der zur Rückgewinnung des Eingangssignals (s₁, g₄) einen Demodulator (14, 15, 31 bis 37) aufweist,
**dadurch gekennzeichnet, daß** der erste Modulator Mittel (2, 17) zur Erzeugung einer Quasi-Dirac-Impulsfolge (s₂, g₁) und ein eingangsseitig mit diesen verbundenes Tiefpaßfilter (3, 18) aufweist, dessen Filterkennlinie kurz vor Erreichen der Grenzfrequenz eine Überhöhung aufweist und die Stoß-Impulsfolge somit in eine Folge von si-Impulsen bzw. Spaltimpulsen (s₃, g₂) transformiert, deren Form durch die si-Funktion si (x) = ^{sinx/}ₓ beschrieben wird, und weiter einen mit dem Ausgang des Tiefpaßfilters verbundenen Amplitudenmodulator (4, 20), der einer Trägerschwingung (f_{T}) eine si-förmige Hüllkurve aufprägt, und ein mit dem Ausgang des Amplitudenrnodulators verbundenes Dispersionsfilter (6; 22, 23) aufweist, der erste Modulator (2 bis 6, 16 bis 24) winketmodulierte Impulse erzeugt,
wobei die Modulation entsprechend einer Modulationscharakteristik erfolgt, welche die zeitliche Änderung der Winkel oder Phasenlage jeweils Während der Dauer eines Impulses bestimmt,
der erste Modulator (16 bis 24) zur Aufnahme des Eingangssignals (g₄) und zur Vorgabe der Modulationscharakteristik in Abhängigkeit von dem Eingangssignal (g₄) einen Steuereingang aufweist erste Modulator oder der (2 bis 6) zur zusätzlichen Modulation der winkelmodulierten Impulse in Abhängigkeit vom Eingangssignal (s₁) einen zweiten Modulator (4) aufweist,
der Empfänger (11 bis 15, 29 bis 37) zur Filterung der senderseitig entsprechend der vorgegebenen Modulationscharakteristik winkelmodulierten Impulse mindestens ein Dispersionsfilter (13, 32, 33) mit einem vorgegebenen frequenzabhängigen differentiellen Laufzeitverhalten aufweist und
das Gruppenlaufzeitverhalten des Dispersionsfilters (13, 32, 33) zur Amplitudenerhöhung des Ausgangssignals (s₉, g₁₄) derart an die senderseitig verwendete Modulationscharakteristik angepaßt ist, daß die spektralen Signalanteile der entsprechend dieser Modulationscharakteristik winketmodulierten Impulse aufgrund der frequenzabhängig unterschiedlichen Signallaufzeit durch das Dispersionsfilter (13, 32, 33) an dessen Ausgang im wesentlichen koinzident und mit einer entsprechenden Amplitudenerhöhung erscheinen.

15. Anordnung nach Anspruch 14, **dadurch gekennzeichnet,**
**daß** der erste Modulator (16 bis 24) eine winkelmodulierte Impulsfolge erzeugt, wobei die Winkelmodulation in Abhängigkeit von dem am Steuereingang anliegenden Eingangssignal (g₄) entweder entsprechend einer vorgegebenen ersten Modulationscharakteristik oder entsprechend einer vorgegebenen zweiten Modulationscharakteristik oder entsprechend einer weiteren Modulationscharakteristik erfolgt, daß der Empfänger (29 bis 37) mindestens zwei parallelgeschaltete Dispersionsfilter (32, 33) aufweist, wobei das unterschiedliche Laufzeitverhalten der beiden Dispersionsfilter (32, 33) und die beiden senderseitig zur Auswahl stehenden Modulationscharakteristiken jeweils paarweise derart aneinander angepaßt sind, daß am Ausgang genau eines dei beiden Dispersionsfilter (32, 33) die spektralen Signalanteile der winkelmodulierten Impulsfolge im wesentlichen koinzident und mit einer entsprechenden Amplitudenerhöhung erscheinen, während bei dem jeweils nicht angepaßten Dispersionsfilter keine Kompression stattfindet.

16. Anordnung nach Anspruch 15, **dadurch gekennzeichnet,**
**daß** der senderseitige erste Modulator (16 bis 24) zur Erzeugung der entsprechend den beiden Modulationscharakteristiken winkelmodulierten Impulsfolge jeweils ein Dispersionsfilter (22, 23) aufweist,
**daß** die in dem ersten Modulator (16 bis 24) angeordneten Dispersionsfilter (22, 23) eingangsseitig über ein steuerbares Schaltelement (21) mit dem Ausgang des Amplitudenmodulators (20) verbunden sind, welche ein Hochfrequenzsignal (g₃) mit einer im wesentlichen si-förmigen Hüllkurve erzeugt, daß das Schaltelement (21) zur Ansteuerung durch das Eingangssignal (g₄) mit dem Steuereingang des Modulators (16 bis 24) verbunden ist.

17. Anordnung nach Anspruch 14, **dadurch gekennzeichnet,**
**daß** der erste Modulator (2 bis 6) winkelmodulierte Impulse erzeugt, wobei die Winkelmodulation, unabhängig vom Eingangssignal (s₁) entsprechend einer vorgegebenen Modulationscharakteristik erfolgt, welche die zeitliche Änderung der Frequenz jeweils während der Dauer eines Impulses bestimmt,
**daß** der senderseitige zweite Modulator (4) zur Aufprägung der in dem Eingangssignal (s₁) enthaltenen Nachricht ein Amplitudenmodulator (4) ist, welcher die Amplitude der winkelmodulierten Impulse in Abhängigkeit von dem Eingangssignal (s₁) vorgibt,
**daß** der Empfänger (11 bis 15) zur Filterung der senderseitig entsprechend der vorgegebenen Modulationscharakteristik winkelmodulierten Impulse genau ein Dispersionsfilter (13) mit einem vorgegebenen frequenzabhängigen differentiellen Laufzeitverhalten aufweist, wobei das Laufzeitverhalten des Dispersionsfilters (13) zur optimalen Amplitudenerhöhung derart an die senderseitig verwendete Modulationscharakteristik angepaßt ist, daß die spektralen Signalanteile jedes winkelmodulierten Impulses am Ausgang des Dispersionsfilters (13) im wesentlichen koinzident und mit einer entsprechenden Amplitudenerhöhung erscheinen,
**daß** dem Dispersionsfilter (13) zur Rückgewinnung der in dem Eingangssignal (s₁) enthaltenen Nachricht ein Detektor (14, 15) nachgeschaltet ist.

18. Anordnung nach Anspruch 14, **dadurch gekennzeichnet,**
**daß** Sender (2 bis 8, 16 bis 26) und Empfänger (11 bis 15, 29 bis 37) zur Ermöglichung eines wechselweisen Sende- und Empfangsbetriebs jeweils korrespondierende, im wesentlichen baugleiche Baugruppen zur Modulation bzw. Demodulation aufweisen, die jeweils mindestens ein Dispersionsfilter (6, 13, 22, 23, 32, 33) enthalten.

19. Anordnung nach Anspruch 14, **dadurch gekennzeichnet,**
**daß** mindestens ein Dispersionsfilter (6, 13, 22, 23, 32, 33) ein Oberflächenwellenfilter ist.

20. Anordnung nach Ansprüche 14, **dadurch gekennzeichnet,**
**daß** der Empfänger (11 bis 15, 29 bis 37) ausgangsseitig ein Meßgerät aufweist zur Messung der Amplitude und/oder der Impulsdauer des Ausgangssignals (s₉, g₁₄),
**daß** im Empfänger (11 bis 15, 29 bis 37) ein Stellglied vorgesehen ist zur Einstellung des frequenzabhängigen Laufzeitverhaltens des Dispersionsfilters (13, 32, 33).

21. Anordnung nach Anspruch 20, **dadurch gekennzeichnet,**
**daß** der Empfänger (11 bis 15, 29 bis 37) eine eingangsseitig mit dem Meßgerät verbundene Steuereinheit aufweist zur Ansteuerung des Stellglieds derart, daß die Amplitude des Ausgangssignals einen Maximalwert bzw. die Impulsdauer des Ausgangssignals einen Minimalwert annimmt.

22. Anordnung nach Anspruch 14, **dadurch gekennzeichnet,**
**daß** der Empfänger eine Rauschunterdrückungsschaltung (38, 47) aufweist, die im wesentlichen aus zwei parallel geschalteten Zweigen besteht, die ausgangsseitig mit den Eingängen eines Subtrahierers (45, 54) verbunden sind und in denen jeweils zwei Dispersionsfilter (39, 44, 40, 43, 48, 52, 49, 53) mit zueinander inversen frequenzabhängigen Laufzeitcharakteristiken in Reihe geschaltet sind, wobei in jedem der beiden Zweige zwischen den beiden Dispersionsfiltern (39, 44, 40, 43, 48, 52, 49, 53) zur Steuerung des Signalflusses ein Steuerelement (41, 42, 50, 51) angeordnet ist, das zur Synchronisation der Signalflußsteuerung mit dem Übertragungstakt mit einer Synchronisierungsschaltung (46, 55 bis 57) verbunden ist.

23. Anordnung nach Anspruch 22, **dadurch gekennzeichnet,**
**daß** das Steuerelement zur Unterbrechung oder zur Freischaltung des Signalflusses ein steuerbares Schaltelement (41, 42) ist, dessen Steuereingang zur zeitgesteuerten Unterbrechung oder Freischaltung des Signalflusses mit der Synchronisierungsschaltung (46) verbunden ist.

24. Anordnung nach Anspruch 22, **dadurch gekennzeichnet,**
**daß** das Steuerelement ein Multiplizierer (50, 51) ist, der eingangsseitig mit dem vorgeschalteten Dispersionsfilter (48, 49) und zur zeitgesteuerten Unterdrückung oder Freischaltung des Signalflusses mit der Synchronisierungsschaltung (55 bis 57) verbunden ist.

## Claims

1. A method for wireless communication with a medical device, in particular one implanted in the human body, whereby a message input signal (s₁, g₄) is subjected to angle modulation, i.e. phase or frequency modulation, in a transmitter (2 to 8, 16 to 26) and travels via a transmission channel to a receiver (11 to 15, 29 to 37),
whereby in the transmitter angle-modulated pulses (s₅, g₅) carrying the information and comprising a frequency spectrum are generated in such a manner that they can be time-compressed in the receiver (11 to 15, 29 to 37) by means of a filter having a frequency-dependent delay time, in particular a dispersion filter (13, 32, 33), in such a manner that pulses having a shorter duration and increased amplitude compared with the emitted pulses are produced, and the pulses at the transmitter side receive at least one part of the information making up the message in impressed form after a further modulation or encoding process of telecommunications engineering or at least one part of the information making up the message is additionally impressed onto the angle modulation,
and whereby in the transmitter first of all a quasi Dirac pulse sequence (s₂, g₁) is approximately generated and is supplied to a low-pass filter (3, 18), the filter characteristic curve of which has a peak shortly before reaching the critical frequency and thus transforms the shock pulse sequence into a sequence of sinc pulses (s₃, g₂), the shape of which is described by the sinc function (si(x)= ^{sinx}/ₓ, which is subsequently passed to an amplitude modulator (4, 20), which impresses a sinc-shaped envelope pulse by pulse onto a carrier frequency (f_{T}), and the signal (s₄, g₃) generated in this manner is fed to a dispersing filter (6; 22, 23), at the output of which a frequency-modulated pulse sequence appears.

2. A method according to Claim 1,
**characterised in that** the pulse sequence is filtered according to a preset filter behaviour, whereby the angle modulation on the transmitter side and the frequency-dependent, differential delay time behaviour of the dispersion filter (13, 32, 33) on the receiver side are matched to one another in such a manner that the spectral signal components of the angle-modulated pulse sequence (s₆) of the output signal (s₉, g₁₄) appear at the output of the dispersion filter (13, 32, 33) substantially coincident and with a corresponding increase in amplitude on account of the frequency-dependent, different signal delay time of the dispersion filter.

3. A method according to Claim 1,
**characterised in that** the further modulation method is a pulse position modulation (PPM) or a pulse code modulation (PCM) or a differential pulse code modulation (DPCM) or a pulse delta modulation (PDM) or a modification of one or more of these modulation methods.

4. A method according to Claim 3,
**characterised in that** the angle modulation and the additional modulation process form independent, orthogonal or approximately orthogonal modulation types.

5. A method according to Claim 1,
**characterised in that** the input signal (g₄) comprises a carrier frequency which is subjected to angle modulation pulse by pulse in the transmitter (16 to 26).

6. A method according to Claim 2,
**characterised in that** the angle-modulated pulses in the receiver (29 to 37) are supplied to at least two dispersion filters (32, 33), whereby the different delay time behaviour of the dispersion filters (32, 33) and the modulation characteristics used at the transmitter side are in each case matched to one another in pairs in such a manner that the spectral signal components of the frequency-modulated pulses appear substantially coincident and with a corresponding increase in amplitude at the output of just one of the two dispersion filters (32, 33), whereas this compression does not take place with the respective other dispersion filter (32, 33).

7. A method according to Claim 1,
**characterised in that** the amplitude of the pulses compressed by the dispersion filter (13) are evaluated by means of a detector (14, 15.

8. A method according to Claim 1,
**characterised in that** the angle modulation takes place in accordance with a preset modulation characteristic, which determines the time change of the phase angle in each case during the duration of a pulse in accordance with a predetermined time-variable sequence,
**in that** in the amplitude demodulator (4) the amplitude of the angle-modulated pulses for impressing the information contained in the input signal (s₁) is preset as a function of the input signal (s₁),
**in that** in the receiver (11 to 15) the angle-modulated pulses are supplied to a dispersion filter (13), the delay time behaviour of which is matched in such a manner to the modulation characteristic of the angle modulation used at the transmitter side with a reverse, time-variable sequence that the spectral signal components of the angle-modulated pulses (s₆) appear substantially coincidentally and with a corresponding increase in amplitude at the output of the dispersion filter (13),
**in that** the amplitude of the pulses compressed by the dispersion filter (13) is evaluated to recover the information contained in the input signal (s₁) by means of a detector (14, 15), in particular an amplitude modulator.

9. A method according to Claim 8,
**characterised in that** during the pulse duration of the pulse-modulated signals, the angle, the frequency or the phase of the carrier frequency steadily changes linearly or non-linearly in accordance with a predetermined profile over time within the pulse duration from a lower frequency or phase position to an upper position or phase position or vice versa, whereby the receiver comprises a correspondingly complementary behaviour of the dispersion filters.

10. A method according to Claim 9,
**characterised in that** the predetermined profile changes within the pulse sequence, in each case in relation to individual pulses to each other, with this change also forming a part of the information.

11. A method according to Claim 1,
**characterised in that** for adjustment a predetermined digital reference signal is transmitted as an input signal (s₁, g₄) to match transmitters (2 to 8, 16 to 26) and receivers (11 to 15, 29 to 37) during a matching operation,
**in that** during that matching operation the amplitude or the pulse duration of the output signal (s₇, g₁₀, g₁₁) of the dispersion filter (13, 32, 33) on the receiver side is measured and the modulation characteristic used at the transmitter side or the frequency-dependent delay time behaviour of the dispersion filter (13, 32, 33) at the receiver side is altered until the pulse duration at the output of the dispersion filters in the receiver assumes a minimum value or the amplitude assumes a maximum value.

12. A method according to Claim 1,
**characterised in that** the signal flow in the receiver is divided into two parallel branches each having two dispersion filters (39, 44, 40, 43) having mutually inverse, frequency-dependent delay time characteristics,
**in that** the signal flow in the two branches is gated or interrupted during a pulse for a predetermined time interval, whereby the interruption or gating takes place synchronously to the transmission clock,
**in that** the two branches are brought together at the output side by a subtractor (45).

13. A method according to Claim 1,
**characterised in that** the frequency range of the carrier signal is in the range of 400 MHz and 1 GHz.

14. A transmitter and receiver configuration for performing the method according to one of the preceding Claims, having a transmitter (2 to 8, 16 to 26) for receiving and transmitting an input signal (s₁, g₄), which comprises a first modulator (2 to 6, 16 to 24) for the angle modulation, i.e. a phase or frequency modulation, of the input signal (s₁, g₄), and also a receiver (11 to 15, 29 to 37), which comprises a demodulator (14, 15, 31 to 37) for recovering the input signal (s₁, g₄),
**characterised in that** the first modulator (2, 17) comprises means for generating a quasi Dirac pulse sequence (s₂, g₁) and a low-pass filter (3, 18) connected thereto at the input side, the filter characteristic curve of which comprises a peak shortly before reaching the critical frequency and thus transforms the shock pulse sequence into a sequence of sinc pulses (S₃, g₂), the shape of which is described by the sinc function si(x)= ^{sinx}/ₓ, and also an amplitude modulator (4, 20), connected to the output of the low-pass filter, which impresses a sinc-shaped envelope on the carrier frequency (f_{T}), and comprises a dispersion filter (6, 22, 23) connected to the output of the amplitude modulator, the first modulator (2 to 6, 16 to 24) generates angle-modulated pulses, whereby the modulation takes place according to a modulation characteristic which determines the temporal change of the angles or phase position in each case during the duration of a pulse,
for receiving the input signal (g₄) and to preset the modulation characteristic as a function of the input signal (g₃) the first modulator (16 to 24) comprises a control input or the first modulation (2 to 6) comprises a second modulator (4) for the additional modulation of the angle-modulated pulses as a function of the input signal (s₁),
for filtering the pulses that are angle-modulated according to the preset modulation characteristic the receiver (11 to 15, 29 to 37) comprises at least one dispersion filter (13, 32, 33) having a preset, frequency-dependent delay time behaviour and for increasing the amplitude of the output signal (s₉, g₁₄) the group delay time behaviour of the dispersion filter (13, 32, 33) is matched to the modulation characteristic used at the transmitter side in such a manner that the spectral signal components of the pulses that are angle-modulated corresponding to this modulation characteristic appear at the output of the dispersion filter (13, 32, 33) substantially coincidentally and with a corresponding increase in amplitude on account of the frequency-dependent different group delay by said dispersion filter.

15. A configuration according to Claim 14,
**characterised in that** the first modulator (16 to 24) generates an angle-modulated pulse sequence, the angle modulation taking place as a function of the input signal (g₄) present at the control input either corresponding to a preset first modulation characteristic or corresponding to a preset second modulation characteristic or corresponding to another modulation characteristic,
**in that** the receiver (29 to 37) comprises at least two parallel-connected dispersion filters (32, 33), wherein the different delay time behaviour of the two dispersion filters (32, 33) and the two modulation characteristics at the transmitter side for selection are in each case matched to one another in pairs in such a manner that at the output of precisely one of the two dispersion filters (32, 33) the spectral signal components of the angle-modulated pulse sequence appear substantially coincidentally and with a corresponding amplitude, while at the non-matched dispersion filter no compression takes place.

16. A configuration according to Claim 15,
**characterised in that** the first transmitter-side modulator (16 to 24) for generating the pulse sequence that is angle-modulated corresponding to the two modulation characteristics in each case comprises a dispersion filter (22, 23),
**in that** the dispersion filters (22, 23) disposed in the first modulator (16 to 24) are connected on the input side via a controllable switching element (21) to the output of the amplitude modulator (20), which generates a high-frequency signal (g₃) having a substantially sinc-shaped envelope,
**in that** the switching element (21) for triggering is connected by the input signal (g₄) to the control input of the modulator (16 to 24).

17. A configuration according to Claim 14,
**characterised in that** the first modulator (2 to 6) generates angle-modulated pulses, the angle modulation taking place independently of the input signal (s₁) corresponding to a preset modulation characteristic, which determines the temporal change of the frequency during the duration of a pulse,
**in that** the transmitter-side second modulator (4) for impressing the information contained in the input signal (s₁) is an amplitude modulator (4), which sets the amplitude of the angle-modulated pulses as a function of the input signal (s₁),
**in that** the receiver (11 to 15) comprises precisely one dispersion filter (13) having a preset, frequency-dependent, differential delay time behaviour for filtering the pulses that are angle-modulated at the transmitter side according to the preset modulation characteristic, wherein for the optimal increase in amplitude the delay time behaviour of the dispersion filter (13) is matched to the modulation characteristic used at the transmitter side in such a manner that the spectral signal components of each angle-modulated pulse appear substantially coincidentally and with a corresponding increase in amplitude at the output of the dispersion filter (13),
**in that** a detector (14, 15) is connected after the dispersion filter (13) to recover the information contained in the input signal (s₁).

18. A configuration according to Claim 14,
**characterised in that** transmitters (2 to 8, 16 to 26) and receivers (11 to 15, 29 to 37), to enable an alternating transmitting and receiving operation, comprise corresponding, substantially identical assemblies for modulation and demodulation, which in each case contain at least one dispersion filter (6, 13, 22, 23, 32, 33).

19. A configuration according to Claim 14,
**characterised in that** at least one dispersion filter (6, 13, 22, 23, 32, 33) is a surface wave filter.

20. A configuration according to Claims 14,
**characterised in that** the receiver (11 to 15, 29 to 37) at the output side comprises a measurement instrument for the measurement of the amplitude and/or the pulse duration of the output signal (S₉, g₁₄),
**in that** in the receiver (11 to 15, 29 to 37) is provided an actuator for the adjustment of the frequency-dependent delay time behaviour of the dispersion filter (13, 32, 33).

21. A configuration according to Claim 20,
**characterised in that** the receiver (11 to 15, 29 to 37). comprises a control unit connected to the measurement device at the input side for triggering the actuator in such a manner that the amplitude of the output signal assumes a maximum value or the pulse duration of the output value assumes a minimum value.

22. A configuration according to Claim 14,
**characterised in that** the receiver comprises a noise suppression circuit (38, 47), which substantially consists of two parallel-connected branches, which at the output side are connected to the inputs of a subtractor (45, 54) and in which in each case two dispersion filters (39, 44, 40, 43, 48, 52, 49, 53) with mutually inverse, frequency-dependent delay time characteristics are connected in series, wherein in each of the two branches between the two dispersion filters (39, 44, 40, 43, 48, 52, 49, 53) for controlling the signal flow is disposed a control element (41, 42, 50, 51), which for the synchronisation of the signal flow control with the transmission clock is connected to a synchronisation circuit (46, 55 to 57).

23. A configuration according to Claim 22,
**characterised in that** the control element for the interruption or the clearing of the signal flow is a controllable switching element (41, 42), the control input of which is connected to the synchronisation circuit (46) for the time-controlled suppression or clearing of the signal flow.

24. A configuration according to Claim 22,
**characterised in that** the control element is a multiplier (50, 51), which at the input side is connected to the preconnected dispersion filter (48, 49) and for the time-controlled suppression or clearing of the signal flow is connected to the synchronisation circuit (55 to 57).

## Revendications

1. Procédé de communication sans fil avec un appareil médical implanté, notamment dans le corps humain,
dans lequel un signal d'entrée d'information (S₁, g₄) est soumis, dans un émetteur (2 à 8, 16 à 26), à une modulation angulaire, c'est-à-dire à une modulation de phase ou de fréquence, et parvient, par l'intermédiaire d'un canal de transmission, à un récepteur (11 à 15, 29 à 37),
dans lequel
dans l'émetteur, des impulsions (s₅, g₉) portant des informations, possédant un spectre de fréquences et modulées selon une modulation angulaire, sont produites de telle sorte que ces impulsions peuvent être comprimées dans le temps dans le récepteur (11 à 15, 29 à 37) à l'aide d'un filtre possédant un temps de propagation qui dépend de la fréquence, notamment un filtre de dispersion (13, 32, 33) de telle sorte qu'il apparaît des impulsions possédant une durée raccourcie par rapport aux impulsions émises et une amplitude accrue, et que sur les impulsions est appliquée, côté émetteur, au moins une partie des éléments constituant les informations, selon un autre procédé de modulation ou de codage de la technique des informations, ou qu'au moins une partie des éléments constituant les informations est appliquée en plus de la modulation angulaire, et dans lequel
dans l'émetteur, tout d'abord approximativement une quasi-suite d'impulsions de Dirac (s₂, g₉) est produite et est envoyée à un filtre passe-bas (3, 18), dont la courbe caractéristique de filtre possède une surélévation peu avant que la fréquence limite soit atteinte, et que la suite d'impulsions de choc est transformée par conséquent en une suite d'impulsions si ou d'impulsions division (s₃, g₂) , dont la forme est décrite par la fonction si si(x) = sinx/x, et envoyée ensuite à un modulateur d'amplitude (4, 20), qui applique une courbe enveloppe de forme si impulsionnelle à l'oscillation de porteuse (f_{T}), et le signal (s₄, g₃) produit de cette manière est envoyé à un filtre de dispersion (6; 22, 23), à la sortie duquel apparaît une suite d'impulsions modulée en fréquence.

2. Procédé selon la revendication 1, **caractérisé en ce que** la suite d'impulsions est filtrée conformément à un comportement prédéterminé du filtre, la modulation angulaire côté émetteur et le comportement de temps de propagation différentiel, qui est présent côté réception et dépend de la fréquence, du filtre de dispersion (13, 32, 33) sont accordées l'un sur l'autre de telle sorte que les composantes spectrales de la suite d'impulsions modulée angulairement (s₆) du signal de sortie (s₉, g₁₄) sur la base du temps de propagation, qui est différent en fonction de la fréquence, du signal du filtre de dispersion (13, 32, 33) apparaissent à la sortie de ce filtre en étant essentiellement coïncidentes et avec un accroissement correspondant de l'amplitude.

3. Procédé selon la revendication 1, **caractérisé en ce qu'**en ce qui concerne la suite du procédé de modulation, il s'agit d'une modulation d'impulsions (PPM) ou d'une modulation par impulsions codées (PCM) ou d'une modulation différentielle à impulsions codées (DPCM), d'une modulation d'impulsions delta (PDM) ou d'une variante d'un ou de plusieurs de ces procédés de modulation.

4. Procédé selon la revendication 3, **caractérisé en ce que** la modulation angulaire et l'autre procédé de modulation forment des types de modulation indépendants l'un de l'autre, orthogonaux ou approximativement orthogonaux.

5. Procédé selon la revendication 1, **caractérisé en ce que** le signal d'entrée (g₄) comporte une fréquence porteuse qui est soumise, dans l'émetteur (16 à 26), respectivement d'une manière impulsionnelle à la modulation angulaire.

6. Procédé selon la revendication 2, **caractérisé en ce que** les impulsions modulées angulairement sont envoyées, dans le récepteur (29 à 37), à au moins deux filtres de dispersion (32, 33), le comportement différent de temps de propagation des filtres de dispersion (32, 33) et les caractéristiques de modulation utilisées sur le côté émission étant adaptées respectivement entre eux par couples, de telle sorte que les composantes spectrales des impulsions modulées en fréquence du signal coïncident pour l'essentiel et apparaissent avec un accroissement correspondant d'amplitude à la sortie uniquement de l'un des deux filtres de dispersion (32, 33), alors que cette compression ne se produit pas dans l'autre filtre respectif de dispersion (32, 33).

7. Procédé selon la revendication 1, **caractérisé en ce que** l'amplitude des impulsions comprimées par le filtre de dispersion (13) est évaluée à l'aide d'un détecteur (14, 15).

8. Procédé selon la revendication 1, **caractérisé en ce que** la modulation angulaire s'effectue conformément à une caractéristique prédéterminée de modulation, qui détermine la variation dans le temps de l'angle de phase respectivement pendant la durée d'une impulsion après le déroulement prédéterminé variable dans le temps,
que dans le modulateur d'amplitude (4), l'amplitude des impulsions modulées angulairement est prédéterminée pour être appliquée aux informations contenues dans le signal d'entrée (s₁), en fonction du signal d'entrée (s₁),
que les impulsions modulées angulairement dans le récepteur (11 à 15) sont envoyées à un filtre de dispersion (13) dont le comportement de temps de propagation est adapté avec un déroulement inverse variable dans le temps, à la caractéristique de modulation angulaire, utilisée sur le côté émission, que les composantes spectrales des impulsions (s₆), modulées angulairement, du signal apparaissent en étant essentiellement coïncidentes et avec un accroissement correspondant en amplitude à la sortie du filtre de dispersion (13),
que l'amplitude des impulsions comprimées par le filtre de dispersion (13) sont évaluées pour la récupération des informations, contenues dans le signal d'entrée (s₁), à l'aide d'un détecteur (14, 15), notamment d'un démodulateur d'amplitude.

9. Procédé selon la revendication 8, **caractérisé en ce que** pendant la durée d'impulsions des signaux modulés selon une modulation d'impulsions, l'angle - la fréquence ou la phase - de la fréquence porteuse varie de façon monotone linéairement ou non linéairement conformément à un profil prédéterminé dans le temps à l'intérieur de la durée de l'impulsion d'une fréquence inférieure ou d'une position de phase inférieure jusqu'à une fréquence supérieure ou une position de phase supérieure ou inversement, le récepteur possédant un comportement complémentaire correspondant des filtres de dispersion.

10. Procédé selon la revendication 9, **caractérisé en ce que** le profil prédéterminé varie à l'intérieur de la suite d'impulsions - respectivement dans le rapport d'impulsions individuelles les unes par rapport aux autres -, cette modification formant également une partie des informations.

11. Procédé selon la revendication 1, **caractérisé en ce que** pour l'adaptation de l'émetteur (2 à 8, 16 à 26) et du récepteur (11 à 15, 29 à 37) pendant un processus d'adaptation pour l'équilibrage, un signal de référence numérique prédéterminé est transmis en tant que signal d'entrée (s₁, g₄),
que pendant le processus d'adaptation, l'amplitude ou la durée des impulsions du signal de sortie (s₇, g₁₀, g₁₁) du filtre de dispersion (13, 32, 33) situé côté réception est mesurée ou la caractéristique de modulation utilisée côté émission ou le comportement de temps de propagation, qui dépend de la fréquence, du filtre de dispersion situé côté réception (13, 32, 33) est modifié jusqu'à ce que la durée des impulsions à la sortie du filtre de dispersion dans le récepteur prenne une valeur minimale ou que l'amplitude prenne une valeur maximale.

12. Procédé selon la revendication 1, **caractérisé en ce que** le flux du signal dans le récepteur est réparti entre deux branches parallèles comportant chacune deux filtres de dispersion (39, 44, 40, 43) possédant des caractéristiques de temps de propagation qui dépendent de la fréquence d'une manière inverse l'une par rapport à l'autre,
que le flux de signal dans les deux branches est transmis ou interrompu respectivement pendant une impulsion pendant un intervalle de temps prédéterminé, l'interruption ou la transmission s'effectuant d'une manière synchrone avec la cadence de transmission, et
que les deux branches sont réunies, côté sortie, par un soustracteur (45).

13. Procédé selon la revendication 1, **caractérisé en ce que** la gamme des fréquences du signal de porteuse se situe dans la gamme de 400 MHz et 1 GHz.

14. Dispositif émetteur et récepteur pour la mise en oeuvre du procédé selon l'une des revendications précédentes, comportant un émetteur (2 à 8, 16 à 26) servant à recevoir et transmettre un signal d'entrée (s₁, g₄), qui comporte un premier modulateur (2 à 6, 16 à 24) pour la modulation angulaire, c'est-à-dire une modulation de phase ou une modulation de fréquence, du signal d'entrée (s₁, g₄) et un récepteur (11 à 15, 29 à 37) qui possède un démodulateur (14, 15, 31 à 37) pour la récupération du signal d'entrée (s₁, g₄), **caractérisé en ce que** le premier modulateur comporte les moyens (2, 17) pour produire une quasi-suite d'impulsions de Dirac (s₂, g₁) et un filtre passe-bas (3, 18) relié à ces moyens et dont la courbe caractéristique de filtre possède une surélévation peu avant que soit atteinte la fréquence limite, et que la suite d'impulsions de choc est transformée par conséquent en une suite d'impulsion si ou d'impulsions de division (s₃, g₂), dont la forme est décrite par la fonction si si(x) = sinx/x, et en outre un modulateur d'amplitude (4, 20) relié à la sortie du filtre passe-bas et qui applique à une oscillation de porteuse (f_{T}) une courbe enveloppe de forme si, et un filtre de dispersion (6; 22, 23) relié à la sortie du modulateur d'amplitude,
que le premier modulateur (2 à 6, 16 à 24) produit des impulsions modulées angulairement, la modulation s'effectuant conformément à une caractéristique de modulation qui détermine la variation dans le temps de l'angle ou de la position de phase respectivement pendant la durée d'une impulsion,
le premier modulateur (16 à 24) servant à recevoir le signal d'entrée (g₄) et à prédéterminer la caractéristique de modulation en fonction du signal d'entrée (g₄) comporte une entrée de commande, ou bien le premier modulateur (2 à 6) pour la modulation supplémentaire des impulsions modulées angulairement possède, en fonction du signal d'entrée (s₁), un second modulateur (4),
le récepteur (11 à 15, 29 à 37) servant à filtrer les impulsions modulées angulairement côté émission conformément à la caractéristique prédéterminée de modulation comporte au moins un filtre de dispersion (13, 32, 33) possédant un comportement de temps de propagation différentiel prédéterminé qui dépend de la fréquence et que le comportement de temps de propagation de groupe du filtre de dispersion (13, 32, 33) est adapté à l'accroissement d'amplitude du signal de sortie (s₉, g₁₄) est adapté à la caractéristique d'évaluation utilisée côté émission de telle sorte que les composantes spectrales des impulsions du signal modulées angulairement conformément à cette caractéristique de modulation, appliquées par le filtre de dispersion (13, 32, 33) apparaissent essentiellement coïncidentes et avec un accroissement correspondant d'amplitude sur la sortie du filtre.

15. Dispositif selon la revendication 14, **caractérisé en ce que** le premier modulateur (16 à 24) produit une suite d'impulsions modulée angulairement, la modulation angulaire s'effectuant en fonction du signal d'entrée (g₄) appliqué à l'entrée de commande, soit conformément à une première caractéristique de modulation prédéterminée, soit conformément à une seconde caractéristique de modulation prédéterminée ou bien conformément à une autre caractéristique de modulation,
que le récepteur (29 à 37) comporte au moins deux filtres de dispersion (32, 33) branchés en parallèle, le comportement différent de temps de propagation des deux filtres de dispersion (32, 33) et les deux caractéristiques de modulation disponibles pour la sélection côté émission sont adaptées respectivement par couples entre eux de telle sorte qu'à la sortie précisément de l'un des deux filtres de dispersion (32, 33), les composantes spectrales de la suite d'impulsions modulée angulairement du signal apparaissent en étant essentiellement coïncidentes et avec un accroissement correspondant d'amplitude alors qu'aucune compression ne se produit dans le cas du filtre de dispersion qui respectivement n'est pas adapté.

16. Dispositif selon la revendication 15, **caractérisé en ce que** le premier modulateur (16 à 24) côté émission comporte, pour la production de la suite d'impulsions modulée angulairement conformément aux deux caractéristiques de modulation, respectivement un filtre de dispersion (22, 23),
que les filtres de dispersion (22, 23), qui sont disposés dans le premier modulateur (16 à 24) sont reliés côté entrée au moyen d'un élément de commutation commandable (21) à la sortie du modulateur d'amplitude (20), qui produit un signal à haute fréquence (g₃) avec une courbe enveloppe essentiellement de forme si,
que l'élément de commutation (21) est relié, pour la commande par le signal d'entrée (g₄), à l'entrée de commande du modulateur (16 à 24).

17. Dispositif selon la revendication 14, **caractérisé en ce que** le premier modulateur (2 à 6) produit des impulsions modulées angulairement, la modulation angulaire s'effectuant d'une manière indépendante du signal d'entrée (s₁) conformément à une caractéristique de modulation prédéterminée, qui détermine la variation dans le temps de la fréquence respectivement pendant la durée d'une impulsion,
que le second modulateur (4) situé côté émission, qui sert à appliquer les informations contenues dans le signal d'entrée (s₁), est un modulateur d'amplitude (4), qui prédétermine l'amplitude des impulsions modulées angulairement en fonction du signal d'entrée (s₁),
que le récepteur (11 à 15) servant à filtrer les impulsions modulées angulairement côté émission conformément à la caractéristique de modulation prédéterminée, comporte précisément un filtre de dispersion (13) possédant un comportement de temps de propagation différentiel prédéterminé, qui dépend de la fréquence, le comportement de temps de propagation du filtre de dispersion (13) étant adapté pour réaliser l'accroissement optimum d'amplitude, pour réaliser, à la caractéristique de modulation utilisée côté émission de telle sorte que les composantes spectrales de chaque impulsion modulée angulairement du signal apparaissent en étant essentiellement coïncidentes et avec un accroissement d'amplitude correspondant sur la sortie du filtre de dispersion (13),
qu'un détecteur (14, 15) est branché en aval du filtre de dispersion (13) pour récupérer les informations contenues dans le signal d'entrée (s₁).

18. Dispositif selon la revendication 14, **caractérisé en ce que** le capteur (2 à 8, 16 à 26) et le récepteur (11 à 15, 29 à 37) comportent, pour permettre un fonctionnement alterné d'émission et de réception, des modules respectifs correspondants, qui possèdent essentiellement le même agencement pour réaliser la modulation et la démodulation et contiennent chacun au moins un filtre de dispersion (6, 13, 22, 23, 32, 33).

19. Dispositif selon la revendication 14, **caractérisé en ce qu'**au moins un filtre de dispersion (6, 13, 22, 23, 32, 33) est un filtre à ondes de surface.

20. Dispositif selon la revendication 14, **caractérisé en ce que** le récepteur (11 à 15, 29 à 37) comporte côté sortie un appareil de mesure pour mesurer l'amplitude et/ou la durée des impulsions du signal de sortie (s₉, g₁₄),
que dans le récepteur (11 à 15, 29 à 37), il est prévu un organe de réglage qui sert à régler le comportement de temps de propagation, qui dépend de la fréquence, du filtre de dispersion (13, 32, 33).

21. Dispositif selon la revendication 20, **caractérisé en ce que** le récepteur (11 à 15, 29 à 37) possède une unité de commande reliée côté entrée à l'appareil de mesure pour commander l'organe de réglage de telle sorte que l'amplitude du signal de sortie prend une valeur maximale ou la durée des impulsions dudit signal de sortie prend une valeur minimale.

22. Dispositif selon la revendication 14, **caractérisé en ce que** le récepteur comporte un circuit de suppression de bruit (38, 47), qui est constitué essentiellement de deux branches parallèles entre elles, qui sont reliées côté sortie aux entrées d'un soustracteur (45, 54) et dans lesquelles respectivement deux filtres de dispersion (39, 44, 40, 43, 48, 52, 49, 53) possédant des caractéristiques de temps de propagation qui sont inverses l'une de l'autre et dépendent de la fréquence, sont branchés en série, auquel cas dans chacune des deux branches est disposé, entre les deux filtres de dispersion (39, 44, 40, 43, 48, 52, 49, 53) pour la commande du flux de signaux, un élément de commande (41, 42, 50, 51) qui est relié, pour la synchronisation de la commande du flux de signaux, à la cadence de transmission au moyen d'un circuit de synchronisation (46, 55 à 57).

23. Dispositif selon la revendication 22, **caractérisé en ce que** l'élément de commande servant à interrompre ou libérer le flux de signaux est un élément de commutation commandable (41, 42), dont l'entrée de commande servant à interrompre ou libérer, d'une manière commandée dans le temps, le flux de signaux est reliée au circuit de synchronisation (46).

24. Dispositif selon la revendication 22, **caractérisé en ce que** l'élément de commande est un multiplieur (50, 51) qui est relié côté entrée au filtre de dispersion branché en amont (48, 49) et, pour la suppression ou la libération, commandée dans le temps, du flux de signaux, est relié au circuit de synchronisation (55 à 57).
